# EUROPEAN PATENT SPECIFICATION

(11) **EP 2 268 669 B1**
(45) Date of publication and mention of the grant of the patent: **18.10.2017**
(21) Application number: 09720973.8
(22) Date of filing: 11.03.2009
(51) Int. Cl.: C07K 16/26, A61K 39/395, A61K 39/00

(54) **Bovine Colostrum comprising anti-insulin antibodies for treating Metabolic Disorder diseases.**
Bovines Kolostrum umfassend anti-Inuslin Antikörper zur Behandlung von metabolischem Syndrom Erkrankungen
Colostrum bovin comprenant des anticorps anti-insuline pour le traitement des maladies du trouble métabolique.

(30) Priority: 13.03.2008 US 36227
(43) Date of publication of application: 05.01.2011
(60) Divisional application: 17172590.6
(73) Proprietor: Immuron Limited, Blackburn North VIC 3130 (AU)
(72) Inventor: ILAN, Yaron, 96400 Jerusalem (IL); LALAZAR, Gad, 90805 Mevasseret Zion (IL); BEN-YAAKOV, Ami, 96348 Jerusalem (IL); ADAR, Tomer, 71700 Modi'in (IL)
(74) Representative: Tranter, Andrew David
(86) International application number: PCT/IL2009/000273
(87) International publication number: WO 2009/113065

(56) References cited:
- WO-A-2005/048935
- DATABASE WPI Thomson Scientific, London, GB; AN 2002-066650 XP002538617 MOROZOW ET AL.: "Composition for treating autoimmune diseases, e.g. diabetes, comprises immunochemical analogs of the antigens that are the target of teh autoimmune reactions" & WO 01/87331 A (MOROZOW ET AL.) 22 November 2001 (2001-11-22)
- EBINA T ET AL: "Treatment of multiple sclerosis with anti-measles cow colostrum" MEDICAL MICROBIOLOGY AND IMMUNOLOGY, SPRINGER, DE, vol. 173, no. 2, 1 September 1984 (1984-09-01), pages 87-93, XP009120465 ISSN: 0300-8584
- SRIRAM S ET AL: "IN-VIVO IMMUNOMODULATION BY MONOCLONAL ANTI-CD4 ANTIBODY II. EFFECT OF T CELL RESPONSE TO MYELIN BASIC PROTEIN AND EXPERIMENTAL ALLERGIC ENCEPHALOMYELITIS" JOURNAL OF IMMUNOLOGY, vol. 141, no. 2, 1988, pages 464-468, XP002538612 ISSN: 0022-1767
- HE F ET AL: "Modulation of human humoral immune response through orally administered bovine colostrum" FEMS IMMUNOLOGY AND MEDICAL MICROBIOLOGY, ELSEVIER SCIENCE B.V., AMSTERDAM, NL, vol. 31, no. 2, 1 August 2001 (2001-08-01), pages 93-96, XP008102760 ISSN: 0928-8244 [retrieved on 2001-07-12]
- SLUKVIN I I ET AL: "B-cell promoting activity of human colostrum" ADVANCES IN EXPERIMENTAL MEDICINE AND BIOLOGY, SPRINGER, US, vol. 371A, 1 January 1995 (1995-01-01), pages 153-157, XP009120570 ISSN: 0065-2598
- JANUSZ M ET AL: "Effect of a proline-rich polypeptide on IgG level in Chickens" ARCHIVUM IMMUNOLOGIAE ET THERAPIAE EXPERIMENTALIS, POLISH ACADEMY OF SCIENCES, WROCLAW, PL, vol. 44, no. 1, 1 January 1996 (1996-01-01), pages 23-26, XP009120466 ISSN: 0004-069X
- T. ADAR ET AL: "Oral administration of immunoglobulin G-enhanced colostrum alleviates insulin resistance and liver injury and is associated with alterations in natural killer T cells", CLINICAL & EXPERIMENTAL IMMUNOLOGY, vol. 167, no. 2, 11 February 2012 (2012-02-11), pages 252-260, XP055079291, ISSN: 0009-9104, DOI: 10.1111/j.1365-2249.2011.04511.x

## Description

### Field of the Invention

The invention relates to an immuno-modulating composition for the treatment and prophylaxis of a Metabolic syndrome disorder using as an active ingredient; bovine colostrum, and a bovine colostrum immunoglobulin preparation comprising anti-insulin antibodies, wherein the bovine colostrum immunoglobulin preparation is obtained from a bovine immunized with insulin. Finding a naturally occurring biochemical defense mechanism capable of controlling immune-related disorders, for example, disorders associated with neoplastic growth, has been the goal of a number of researchers for many years. Use of the immune system against malignant tumors forms the basis for many anti-cancer strategies. For example, U.S. Pat. No. 5,980,896 describe certain antibodies, antibody fragments and antibody conjugates and single-chain immunotoxins directed against human carcinoma cells. Conventional anti-tumor immuno-therapies rely on antibody-antigen recognition chemistry, and on targeting of antibodies against various antigenic features of tumor cells in order to trigger destruction of the tumor cells by the body's immune system or to target the tumor cells with antibody conjugates of various cytotoxic or chemotherapeutic agents. In practice, however, tumors *in vivo* have generally not been found to be very immunogenic and in many instances appear to be capable of evading the body's immune response. Today a great deal of anti-cancer work is directed at finding ways of increasing the immunogenicity of a tumor cell *in vivo*, by modulating and preferably, stimulating the subject's immune response against the cancer. Many studies have attempted use of IgG as passive immunity or stimulation of natural IgG production to restrict tumor growth.

For decades, various attempts have been made to obtain increased secretion of immunogen-specific antibodies via the mammary gland of farm animals. Such attempts are aimed at production of large quantities of immunogen-specific antibodies via milk. The antibody levels in mature milk, however, still remain lower (approximately an order of magnitude) when compared to those that can be achieved in colostrum.

Colostrum (also known as first milk) is a form of milk produced by the mammary glands in late pregnancy and the few days after birth. In humans it has high concentrations of nutrients and antibodies, but is small in quantity. Colostrum is high in carbohydrates, protein, mineral salts, vitamins and immunoglobulin. It also contains various floating cells such as granular and stromal cells, neutrophils, monocyte/macrophages and lymphocytes and includes growth factors, hormones and cytokines.

Leukocytes are also present in colostrum in large numbers which enable protection against viruses and bacteria. Colostral leukocytes enhance passive immunity of neonatal calf, especially in regard to antibodies and immunoglobulin classes which are essential for intestinal immunity.

The large numbers of secretory antibodies found in the colostrum help protect the mucous membranes in the throat, lungs, and intestines of the newborn. Bovine colostrum (BC) contains three major classes of immunoglobulins: IgG, IgM and IgA.

As indicated above, colostrum is quite a unique product that arises from a distinct physiological and functional state of the mammary gland. In ruminants, the principal compositional difference between colostrum and mature milk is the very high content of bioactive components such as lactoferrin and immunoglobulins [Tarbell, K.V. et al. J. Exp. Med. 199:1467-77 (2004); Bluestone, J.A. and Tang, Q. J. Autoimmun 24:55-62 (2005); Putnam, A.1. et al J.Autoimmun. 24:55-62 (2005)], of which IgG class makes up 80-90%.

Various factors have been isolated and characterized from mammalian colostrum. In 1974, Janusz et al. [Janusz, FEBS Lett. 49:276-279 (1974)] isolated a proline-rich polypeptide (PRP) from ovine colostrum. It has since been discovered that mammals other than sheep have analogues of PRP as a component of their colostrum. PRP has since been called Colostrinin and is tentatively identified as a new class of cytokine.

Colostrum further comprises chemotactic activators of the immune system known as alarmins. The mechanisms by which multicellular organisms respond to infections and tissue injury, as well as restore tissue homeostasis are known as "the inflammatory response,". The initiation of an appropriate inflammatory response requires the recognition of exogenous as well as endogenous danger signals- molecules that alert the innate immune system and trigger defensive immune responses. Endogenous molecules which initiate inflammatory responses by interaction with signaling receptors are known as endokines and/or alarmins. These potent immunostimulants are rapidly released following pathogen challenge and/or cell death, recruit and activate antigen-presenting cells which are critical for mounting an immune response, and include defensins, cathelicidin, eosinophil-derived neurotoxin, and high-mobility group box protein 1 (HMGB1).

The immunization of an animal such as a cow with specific antigens enables the production and harvest of specific antibodies that may be used for modulation of an immune response and thereby in the treatment of immune-related disorders. Accordingly, this method serves as an easy and safe means for generating antigen-specific antibodies and immune adjuvants.

Several previous patents and patent applications by part of the present inventors (ANADIS LTD.), described the use of specific bacterial pathogens antibodies, obtained from bovine colostrum for the passive treatment of infectious diseases. For example, WO 04/078209 by part of the present inventors describes compounds and compositions for treatment or prophylaxis of gastrointestinal disorders prepared by immunizing a host animal with a vaccine comprising one or more cell wall antigens of enteric bacteria, specifically, gram negative bacteria. The hyper immune material produced is in the form of tablets for oral administration. WO 03/097094 describes the use of a hyper immune colostrum in the production of antibodies (whole IgG), or F(ab')₂ antibodies fragments, conjugated with mammalian colostrums and colostrums extracts, for intranasal administration aimed at the prevention of symptoms arising from the presence of air-borne pathogenic bacteria.

These and similar publication of the present inventors give a strong indication as to the great potential of using colostrum-derived immunoglobulin preparation for passive immunization. Mucosal tolerance is considered as an attractive approach for the treatment of autoimmune and inflammatory diseases due to the lack of toxicity, ease of administration, and antigen-specific mechanism of action [Wershil, B.K. and Furuta, G.T. J. Allergy Clin. Immunol. 121:S380-3; quiz S415 (2008); Faria, A.M. and Weiner, H.L. Clin. Dev. Immunol. 13:143-57 (2006)]. Hence, major attempts were made to generate stable colostrum-derived products suitable for oral and nasal administration. For Example, WO 95/08562 by part of the inventors, describes the method of obtaining high purity immunoglobulins from antibody rich colostrum and the possibility of compressing these colostral-antibodies into a tablet form without substantial loss of activity. Specific antibodies are obtained by immunization of a mammal with specific antigens against enterotoxic bacteria such as *E. coli*, *Salmonella* and *Shigella.* Finally, WO 06/053383 by part of the inventors, describes a carboxylic acid and alkalizing moieties which confer upon a bioactive agent composition of a hyper immune colostrum, lactoferrin or lactoferracin, stability under a wide variety of gastric pH values, and WO 03/080082 by part of the inventors, describes a method of improving the viability of a labile bioactive substance, preferably immunoglobulins or fragments thereof or enzymes, in a gastric environment, comprising forming a mixture of the bioactive substance and mammalian colostrum and colostrums extracts. This conjugation protects the antibodies or antibodies fragments from the proteolysis occasioned by enzyme or low pH conditions and preserve their function in the stomach or rumen or other hostile environment.

Regulatory T (Tregs) cells are specialized subpopulation of T cells that act to suppress activation of the immune system and thereby maintain immune system homeostasis and tolerance to self-antigens, and limit chronic inflammatory diseases [Vignali, D.A. et al. Nat. Rev. Immunol. 8:523-32 (2008)]. Induction of Tregs is accepted as a main pathway for immunotherapy in immune mediated disorders [Lopez-Diego, R.S. and Weiner, H.L. Nat. Rev. Drug Discov. 7:909-25 (2008)]. Tregs promotion occurs without systemic immuno-suppression, making this intervention safe and with a low rate of side effects.

Oral administration of CD3-specific antibodies was recently shown to exert an immune modulatory effect by promoting Tregs, and specifically, CD4+CD25-LAP+ Tregs, which function in vitro and in vivo through a TGF-β-dependent mechanism [Ochi, H. et al. Nat. Med. 12:627-35 (2006)]. Given orally, monoclonal anti-CD3 antibodies, by inducing Tregs, suppress experimental allergic encephalomyelitis, diabetes and lupus [Ochi (2006) ibid. Wu, H.Y. et al. J. Immunol 181:6038-50 (2008); Chen, M.L. Y.et al. J. Immunol. 180:7327-37 (2008); Ishikawa, H. et al. Diabetes 56:2103-9 (2007); Gandhi, R. et al. J. Immunol. 178:4017-21 (2007)]. CD4+ T cells stimulated with anti-CD3 markedly suppressed the proliferation and cytokine production of autologous peripheral blood monocytes [Abraham, M. et al. J. Autoimmun. 30:21-8 (2008)]. These Tregs were not induced by incubation with isotype control antibody or by a combination of anti-CD3 with high doses of anti-CD28. These data support the existence of human T cells with strong regulatory properties that are induced by antibodies and that appear to act in a non-HLA restricted fashion by affecting APCs [Abraham (2008) ibid.].

Exactly how IgG crosses epithelial barriers to function in mucosal immunity remains unknown [Spiekermann, G.M. et al. J. Exp. Med. 196:303-10 (2002)], although it was previously suggested to be performed mainly by specialized antigen-presenting cells (APCs), such as dendritic cells (DCs). Innate immune recognition by stromal cells has important implications for regulating the mucosal homeostasis and for the initiation of innate and adaptive immunity [Fritz, J.H. et al. Trends Immunol. 29:41-9 (2008); Dahan, S. et al. Immunol. Rev. 215:243-53 (2007)]. Immunization with islet auto-antigens induce islet antigen-specific Tregs and prevent type 1 diabetes mellitus (T1DM) [Von, Herrath. et al. J. Clin. Invest. 98:1324-31 (1996)]. These antigen-specific Tregs act as bystander suppressors of heterologous auto reactive immune responses [Homann, D. et al. J. Immunol. 163:1833-8 (1999); Homann, D. et al. Immunity 11:463-72(1999)].

Combination treatment with anti-CD3-specific antibodies and a proinsulin peptide reversed recent-onset diabetes in two murine diabetes models, exhibiting much higher - efficacy than monotherapy with anti-CD 3 or antigen alone. The combination therapy increased the level of CD4+CD25+Foxp3+ Tregs and enhanced their proinsulin-specific IL-10, IL-4, and TNF-β production. In addition, combination therapy suppresses auto-aggressive CD8 responses [Bresson, D. et al. J. Clin. Invest. 116:1371-81 (2006)]. Upon immunization with islet auto-antigens, combination therapy with anti-CD3 expands islet-specific Tregs more forcefully.

Chronic inflammation is an important pathophysiological mechanism in type 2 diabetes mellitus (T2DM) [Donath, M.Y. and Halban, P.A. Diabetologia 47:581-9 (2004); Donath, M.Y.et al. J. Mol. Med. 81:455-70 (2003); Bloomgarden, Z.T. Diabetes Care 28:2312-9 (2005); Tan, K.C. et al. Diabetes Care 27:223-8 (2004)]. In the process of inflammation, macrophages and the endothelium contribute to increased serum levels of different cytokines including IL-1β, IL-6, and TNF in T2DM patients [Pickup, J.C. and Crook, M.A. Diabetologia 41:1241-8 (1998)]. The metabolic syndrome is currently recognized as a pro-inflammatory and prothrombotic condition, with increased C-reactive protein (CRP) and interleukin (IL)-6 [Ruth, M.R. and Proctor, S.D. Int. J. Obes. (Lond) 33:96-103 (2009); Dandona, P. et al. Trends Immunol. 25:4-7 (2004); Hotamisligil, G.S. Int. J. Obes. Relat. Metab. Disord. 27 Suppl 3:S53-5 (2003)].

Adipose tissue is increasingly recognized as a metabolically active endocrine organ with multiple functions beyond its lipid storage capability [Permana, P.A. et al. Methods Mol. Biol. 456:141-54 (2008)]. Adipose tissue has an active role in the development and maintenance of insulin resistance. In obese individuals, adipose tissue releases increased amounts of non-esterified fatty acids, glycerol, hormones, pro-inflammatory cytokines and other factors that are involved in the development of insulin resistance [Hotamisligil, G.S. Nature 444:860-7 (2006); Kahn, S.E. et al. Nature 444:840-6 (2006)]. When insulin resistance is accompanied by dysfunction of pancreatic islet beta-cells, the cells that release insulin, failure to control blood glucose levels results. Abnormalities in beta-cell function are therefore critical in defining the risk and development of type 2 diabetes. A crosstalk between lymphocytes and adipocytes was suggested important in this setting [Poggi, M. et al. Diabetologia (2009)].

Various constituents of the adipose tissue, such as mature adipocytes and stromal vascular cells, have distinct functions [Permana (2008) ibid.]. They express and secrete different kinds of bioactive molecules collectively called adipokines. Altered adipokine secretion patterns characterize obesity and insulin resistance, which are major risk factors for type 2 diabetes mellitus.

Alterations in Tregs have been associated with type 1 diabetes mellitus (T1DM) [Yan, Y. et al. Int. J. Immunopathol. Pharmacol. 21:767-80 (2008); Lazarski, C.A. Immunity 29:511-512 (2008); Manirarora, J.N. et al. PLoS ONE 3:e3739 (2008); Lawson, J.M. et al. Clin. Exp. Immunol. 154:353-9 (2008)]and with the development of type 2 diabetes mellitus (T2DM) [Sharif, S. et al. Ann. N.Y. Acad. Sci. 958:77-88 (2002)]. CD4+CD25+ Tregs were shown to modulate the activity of the insulin receptor in an animal model of T2DM [Zhao, H. et al. Diabetes 56:1210-8 (2007)]. In patients with T2DM, exercise-induced-increase in CD4+CD25+ Tregs correlated with decreased hemoglobin A1C serum levels [Zhao (2007) ibid.].

The present invention now demonstrate the use of mammalian colostrum-derived immunoglobulin preparations as immuno-modulators capable of modulating immune-regulatory cells, specifically, regulatory T cells. Such modulation may results for example, in modulation of the Th1/Th2, Trl/Th3 cell balance, which governs the immune response. These effects upon the immune system enable the use of such colostrums-derived preparations for the treatment of immune-related disorders in an active, as well as passive manner. Moreover, these immuno-globulin preparations and compositions thereof may be directed to antigens specific for a certain immune-related disorders or alternatively, may modulate the immune-response in a disease non-specific manner by inducing specific cells or parts of the immune system in a non specific way, including an immune bystander effect, or non disease target antigen.

As described above, the use of antibodies for immuno-modulation have been previously shown by others, for example, WO 2005/048935 [Weiner et al.], describes the immunomodulatory effect of an anti CD3 antibody on autoimmune disorders. More specifically, this publication shows that oral and mucosal administration of anti-CD 3 antibody suppresses experimental allergic encephalomyelitis (EAE), delays allograft rejection in a dose-dependent fashion, reduces the severity of arthritis and prevents the onset of diabetes in NOD mouse model. In contrast, the colostrum-derived immunoglobulin preparations of the present invention are directed towards antigens derived from samples obtained from diseased subjects, or disease-related antigens, (such as insulin, for treating diabetes type 2), and therefore activate regulatory T cells specific for a certain disease.

Thus, the colostrum-derived immunoglobulin preparation of the invention may provide specific and optionally, tailored compositions for efficient treatment of a certain immune-related disorder, by an active manner as mediated by regulatory T cells.

It is therefore an object of the invention to provide immunomodulatory compositions comprising colostrum-derived immunoglobulin preparation, and uses thereof in methods of preventing and/or treating immune-related disorders, specifically, Metabolic Syndrome These and other objects of the invention will become clearer as the description proceeds.

### Summary of the Invention

The scope of the invention is defined by the claims. In a first aspect, the invention relates to an immuno-modulating composition for the treatment and prophylaxis of a Metabolic Syndrome. More specifically, such composition comprises as an active ingredient bovine colostrum, and a bovine colostrum immunoglobulin preparation comprising anti-insulin antibodies, wherein the bovine colostrum immunoglobulin preparation is obtained from a bovine immunized with insulin and modulates regulatory T cells, thereby activating or inhibiting an immune response. The immunoglobulin preparation of the invention is capable of recognizing and binding at least one antigen specific for the disorder and modulating immune-regulatory cells, specifically, regulatory T cells. Such modulation may lead for example, to modulation of the Th1/Th2, Trl/Th3 cell balance thereby activating or inhibiting an immune response specifically directed toward said disorder. It should be noted that this immuno-modulatory effect may be mediated by activation or promotion of specific subsets of regulatory cells, or antigen presenting cells, via direct or indirect activation. It should be further appreciated that the colostrum-derived immuno-globulin preparation of the invention may act in an antigen specific and non specific manner, by working against bystander antigens, or by being directed towards non associated antigens.

These and other aspects of the invention will become apparent by the hand of the following figures.

### Brief Description of the Figures

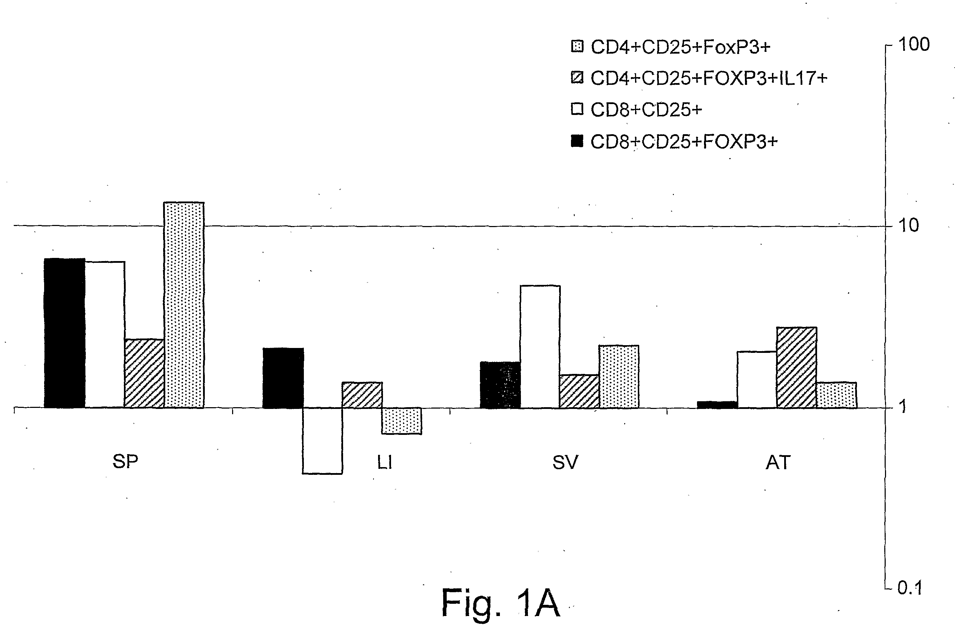

**Figure 1A-1B****: *Effect of oral administration of colostrum-enriched antibodies on regulatory T cells distribution***
   FACS analysis was performed on lymphocytes isolated from adipose tissue, adipose tissue associated stromal vasculature, liver and spleen of mice treated with anti insulin antibodies (group B), mice treated with purified anti insulin antibodies (group D) and untreated controls (group G).
   **Fig. 1A****.** The ratio of Tregs as determined by the FACS analysis for group B versus control group G was calculated for the four types of Tregs CD4+CD25+FoxP3+, CD4+CD25+FoxP3+IL17+, CD8+CD25+, and CD8+CD25+FoxP3+.
   **Fig. 1B**. The ratio of Tregs determined by the FACS analysis for group D versus control group G was calculated for the four types of Tregs CD4+CD25+FoxP3+, CD4+CD25+FoxP3+IL17+, CD8+CD25+, and CD8+CD25+FoxP3+.
   Abbreviations: AT (adipose tissue), SV (stromal vasculature), SP (spleen), LI (liver).
**Figure 2A-2D****: *Effect of oral administration of colostrum-enriched antibodies on insulin resistance***

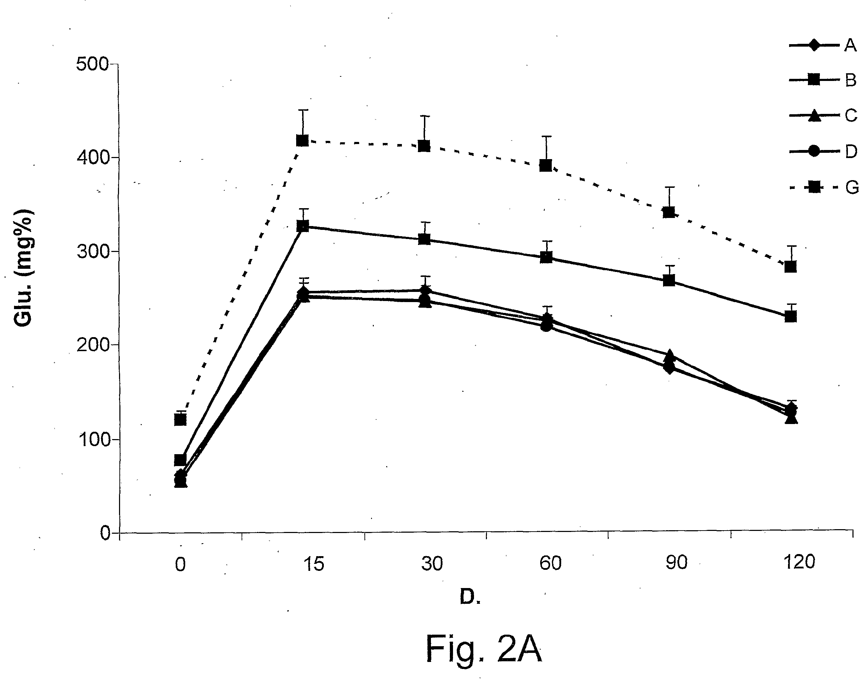

   **Fig 2A****.** Mice treated with anti insulin antibodies in low and high doses (groups A and B, respectively), mice fed with purified anti insulin antibodies (groups C and D, respectively), and untreated mice (group G) underwent glucose tolerance test. The decrease was significant for group A-D versus G (p<0.005)

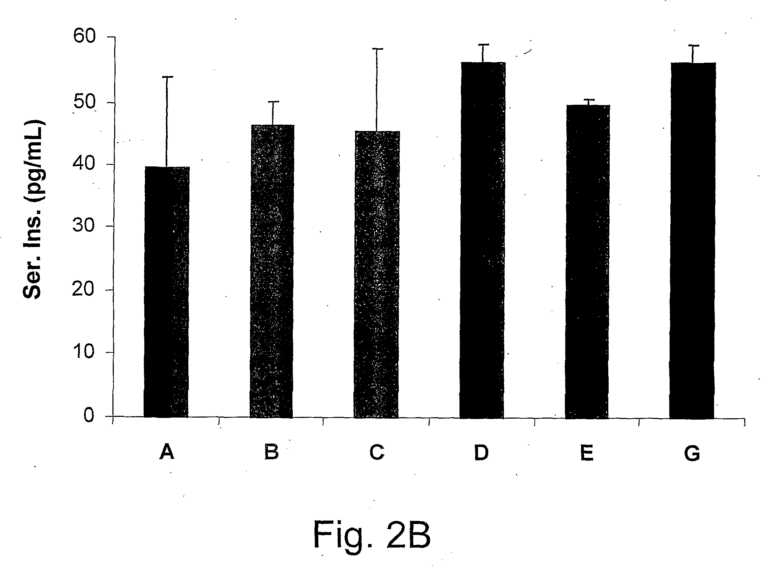

   **Fig 2B****.** Levels of fasting serum insulin were teased in mice of groups A-E and G following four weeks of feeding. The decrease was significant for group A-C versus G (p<0.005)

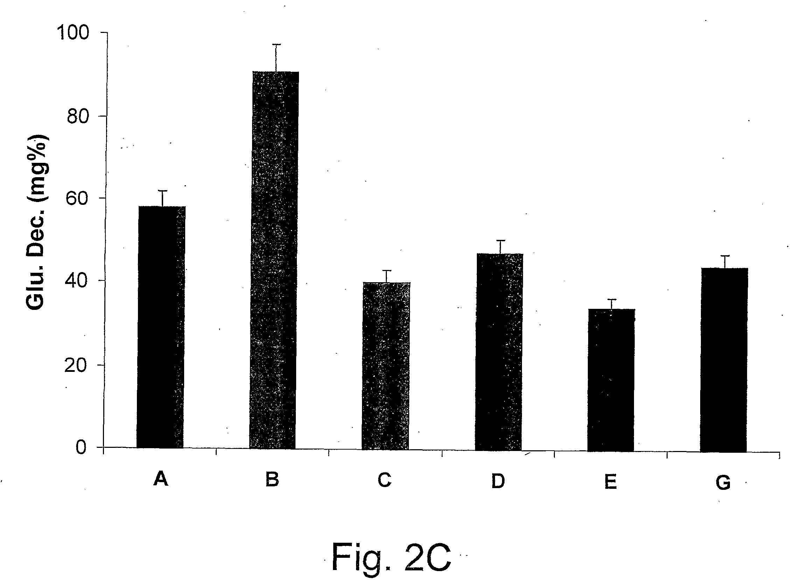

   **Fig 2C****.** Levels of fasting serum glucose were measured in the mice from groups A-E and G on a weekly basis, and the mean decrease over four weeks of treatment was calculated. The mean decrease was significantly higher for mice in groups A and B, vs. C-E and G (p<0.005).

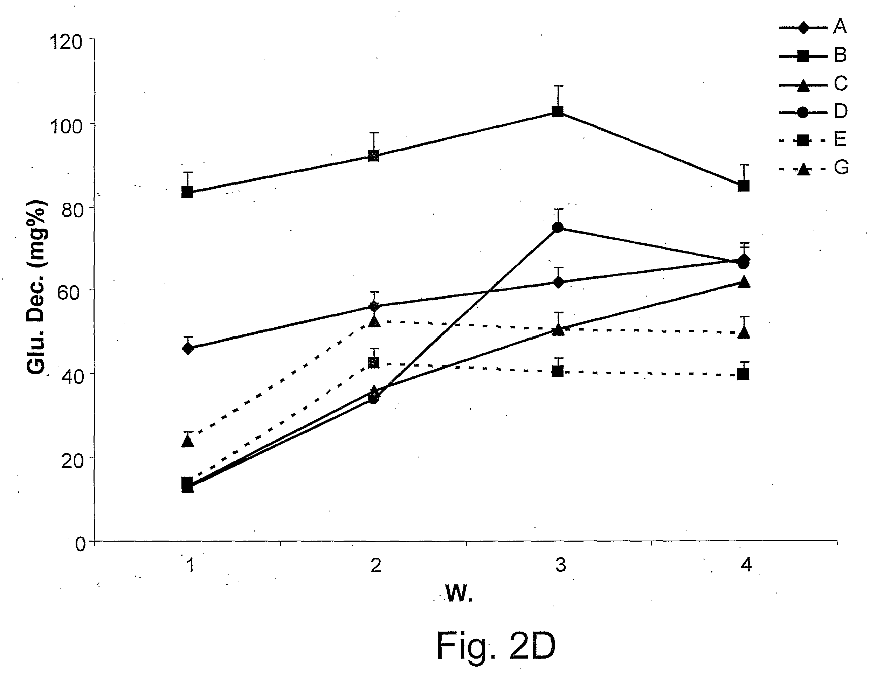

   **Fig 2D****.** The decrease in fasting serum glucose levels in mice from groups A-E and G per week is shown. A significantly higher decrease was noted throughout the study from mice in group B versus all other groups (p<0.005), and for mice in group A versus C, D, E and G on weeks 1, 3 and 4 (p<0.005).
   Abbreviations: D (days), W (weeks), Glu (glucose), Ser (serum), Ins (insulin), Dec (decrease).

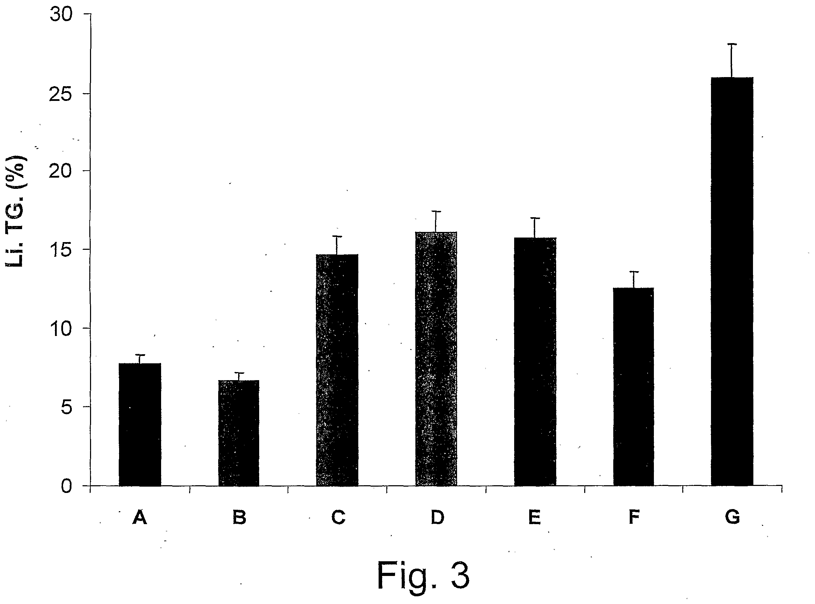

**Figure 3****: *Effect of oral administration of colostrum-enriched antibodies on liver injury***
   Liver triglyceride content was calculated at the end of the study on all treated and control groups. The decrease was significant for group A -F vs. G (p<0.005), and for group A and B vs. C, D, E, and F (P<0.001). Abbreviations: Li (liver), TG (triglycerides).

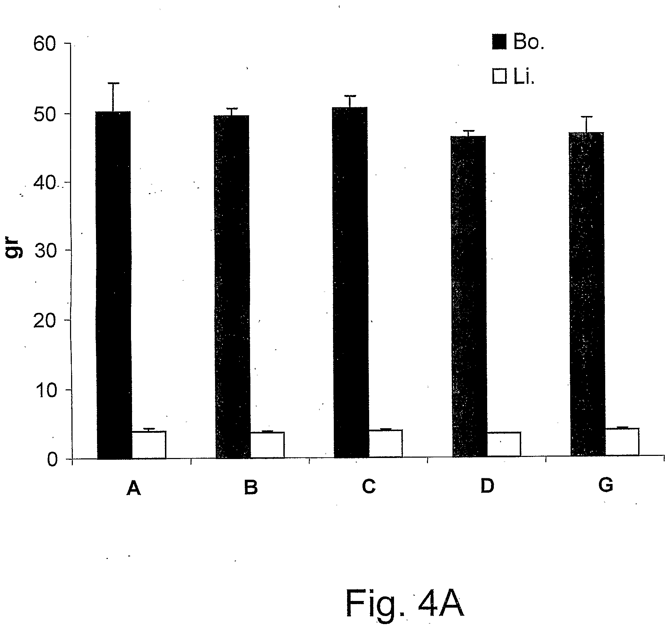

**Figure 4A-4B****: *Effect of oral administration of colostrum-enriched antibodies on body weight and liver weight***
   **Fig 4A****.** Body and liver of mice from groups A-D and G were weighed at the end of the study. No significant differences were noted between the groups.

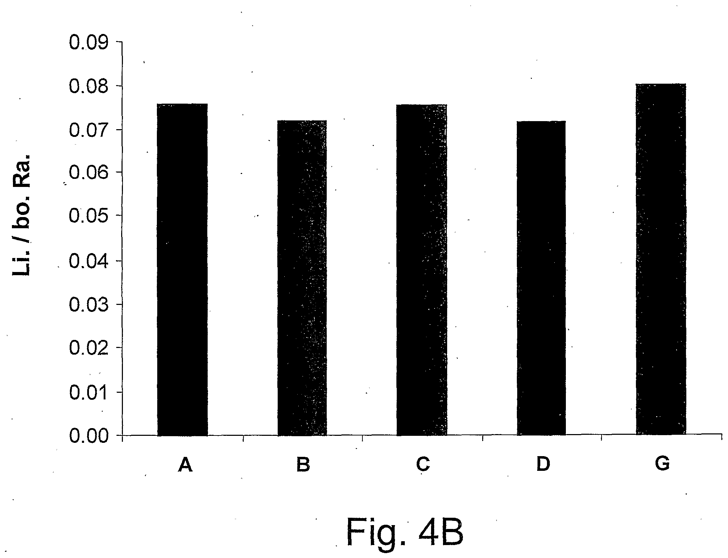

   **Fig 4B****.** Body vs. liver weight ratio was calculated for each group at the end of the study. No significant differences were noted between the different groups.
   Abbreviations: LI (liver), BO (body), RA (ratio).

### Detailed Description of the Invention

The scope of the invention is defined by the claims. A productive immune response results from the effective integration of positive and negative signals that have an impact on both innate and adaptive immune cells. When positive signals dominate, cell activation and pro-inflammatory responses ensue, resulting in the elimination of pathogenic microorganisms, viruses as well a transformed cell. In the absence of such productive stimulation, cell activation is blocked and active anti-inflammatory responses can occur. Modulation of this binary system occurs through the action of cytokines, downstream signaling pathways and cell-cell contact. The perturbation of these thresholds can result in aberrant responses that are either insufficient to deal with pathogenic microorganisms or result in the loss of tolerance and the induction of autoimmune responses. The present invention shows an immunomodulatory effect of a colostrum-derived immunoglobulin preparation that may act in an active manner for the treatment of immune-related disorders.

Regulatory T cells (Tregs) are increasingly recognized as an important immunomodulatory component of the adaptive immune system. Immune dysregulation may lead to chronic inflammation as a trigger for chronic insulin insensitivity. The present invention shows in a particular example, that oral administration of colostrum-derived anti insulin antibodies promote Tregs in adipose tissue and in adipose tissue associated stromal vasculature. These alterations are associated with alleviation of the Metabolic Syndrome and the associated liver injury in the ob/ob mice model. Therefore, the present invention provides as a novel therapeutic composition for the alleviation and treatment of the Metabolic Syndrome. Previous studies have shown that feeding of non specific antibodies, anti-CD3, is an effective method for the induction of Tregs [Ochi (2006) ibid. Ishikawa (2007) ibid.; Abraham (2008) ibid.; Ochi, H. et al. J. Neurol. Sci. 274:9-12 (2008); Zhang, X. et al. Int. Immunol. 18:495-503 (2006)]. In contrast, the data provided herein shows for the first time that the use of antigen specific antibodies can induce Tregs in an organ specific manner. Specifically, the invention shows that feeding of anti insulin antibodies promoted Tregs in the adipose tissue and SV (stromal-vascular cells). Both, CD4+CD25+FoxP3+ and CD8+CD25+FoxP3+ lymphocyte subsets are promoted in these organs. Although both types of Tregs were previously suggested to play a role in diabetes [Bresson, D. et al. J. Clin. Invest. 116:1371-81 (2006), Wang, R. Immunology 126:123-31 (2009), Tarbell, K.V. et al. J. Exp. Med. 199:1467-77 (2004); Bluestone (2005) ibid.; Putnam (2005) ibid.; James (2007) ibid.], adipose tissue and SV-derived cells were not previously described to be associated with alleviation of the Metabolic Syndrome.

Further analysis reveled that the adipose tissue ob/ob mice fed with colostrum enriched with anti insulin antibodies had a significantly higher proportion of CD4+CD25+Foxp3+ and CD8+CD25+ lymphocytes in their adipose tissue and SV. This data suggest that the adjuvant effect of the colostrum itself has an effect on the tissue distribution of Tregs.

Various constituents of the adipose tissue, such as mature adipocytes and stromal vascular cells, have distinct functions [Permana (2008) ibid.]. They express and secrete different kinds of bioactive molecules collectively called adipokines. Altered adipokine secretion patterns characterize obesity and insulin resistance, which are major risk factors for type 2 diabetes mellitus. Regional and genotypic differences are present in stromal-vascular cells from obese and lean Zucker rats [Turkenkopf, I. J. et al. Int. J. Obes. 12:515-24 (1988)]. Gene expression profiling using DNA microarrays showed differences between adipose tissue, adipocytes, and stromal vascular cells [Permana (2008) ibid.]. The present invention further supports this notion, showing that the distribution of Tregs in these tissues is important in the metabolic syndrome.

In addition to the increase in CD4+CD25+ and CD8+CD25+ Tregs, the invention shows an increase in a unique population of cells, CD4+CD25+FOXP3+IL17+, a subset that was promoted both in the adipose tissue and SV by oral administration of colostrum-derived anti insulin antibodies.

The invention further shows that the promotion of Tregs in the adipose tissue and SV by administration of anti insulin antibodies is associated with insulin resistance alleviation. This is demonstrated by glucose levels and glucose tolerance tests, indicating a decrease both in gluconeogensis (decreased fasting glucose) and in insulin resistance (reduced GTT values and AUC). In addition, the inflammatory liver damage is alleviated by the present invention, as manifested by a decrease in liver enzymes, and hepatic fat accumulation.

As described above, the invention shows that oral administration of colostrum-enriched with anti insulin antibodies can serve as a mean to promote Tregs in the adipose tissue and the adipose tissue associated stromal vasculature. Previous studies showed that Colostrum-derived exosomes inhibit anti-CD3-induced IL-2 and IFN-gamma production from allogeneic and autologous PBMC [Nagatomo, T. et al. Clin. Exp. Immunol. 138:47-53 (2004); Admyre, C. et al. J. Immunol. 179:1969-78 (2007)], and an increased number of Foxp3+CD4+CD25+ Tregs were observed in PBMC incubated with milk vesicle preparations [Awasthi, A. et al J. Clin. Immunol 28:660-70 (2008)]. CD25 expression in PBMC was enhanced by pretreatment with colostrum derived IL-1β, TNF- β and IFN-γ [Harlan, D.M. and Von Herrath, M. Nat. Med. 11:716-8 (2005)]. Oral administration of bovine colostrum (BC) can also affect the local immunity in the intestine altering the T cell polarization. The CD21+/CD3+ cells populations of the ileal Peyer's patch (iPP) are markedly affected by BC. Exposure to BC increased the percentage of cells expressing CD11a, CD11c and CD43, but decreased the percentage of cells expressing CD62L relative to freshly isolated PBMC, facilitating lymphocyte trafficking.

Furthermore, the present invention shows that the specificity of the antibody fed is essential for Treg accumulation. Administration of non anti specific antibodies enriched colostrum derived solution (NAIS-CDS), containing anti E. coli antibodies proved to be less efficient than anti insulin antibodies in promotion of Tregs in adipose tissue and SV, and as a result less efficient in improving insulin resistance.

The invention also presents the additive value of the colostrums-derived adjuvants by its effect on the distribution of Tregs, and by a more profound effect noted on fasting glucose and insulin levels. Several proteins were identified in breast milk as involved in host defense [Kahn, S.E. et al. Nature 444:840-6 (2006)], including high concentrations mediators of the innate immune system [Poggi, M. et al. Diabetologia (2009)]. Among these mediators are multiple defensin proteins, sphingolipids, osteopontin, exosomes, TLRs, cathelicidin, eosinophil-derived neurotoxin, and high-mobility group box protein 1, and LL-37 [Poggi (2009) ibid.; Nagatomo, T. et al. Clin. Exp. Immunol. 138:47-53 (2004); Admyre, C. et al. J. Immunol. 179:1969-78 (2007); Oppenheim, J. J. and Yang, D. Curr. Opin. Immunol. 17:359-65 (2005)]. These can activate the innate and adaptive immune systems. Some of these proteins are also termed 'alarmins', in recognition of their role in mobilizing the immune system [Oppenheim (2005) ibid.]. Alarmins have both chemotactic and activating effects on APCs, and can thus amplify innate and Ag-specific immune responses to danger signals [Yang, D. et al. J. Immunol. 173:6134-42 (2004); Oppenheim, J. J. et al. Adv. Exp. Med. Biol. 601:185-94 (2007)]. BC (bovine colostrum) contains high levels of β-glycosphingolipids (BGS) [Martin, M.J. et al. Lipids 36:291-8 (2001); Sala-Vila, A. et al. Nutrition 21:467-73 (2005); Van, Y.H. et al. Diabetes 58:146-55 (2009); Nagatomo, T. et al. Clin. Exp. Immunol. 138:47-53 (2004)], the composition of which can determine the effect of APCs or other components of the gut-immune system [Novak, J. et al. Int. Rev. Immunol. 26:49-72 (2007); Nowak, M. and Stein-Streilein, J. Int. Rev. Immunol. 26:95-119 (2007); Nikoopour, E. and Schwartz, J.A. Inflamm. Allergy Drug Targets 7:203-10 (2008); Admyre, C. et al. J. Immunol. 179:1969-78 (2007); Oppenheim, J. J. and Yang, D. Curr. Opin. Immunol. 17:359-65 (2005); Yang, D. et al. J. Immunol. 173:6134-42 (2004); Oppenheim, J. J. et al. Adv. Exp. Med. Biol. 601:185-94 (2007)]. Some of these mediators can serve as mucosal adjuvants, enhancing the cross talk between subsets of APCs and Tregs in the bowel mucosa [Vignali, D.A. et al. Nat. Rev. Immunol. 8:523-32 (2008); Margalit, M. et al. J. Pharmacol. Exp. Ther. 319:105-10 (2006); Godfrey, D.I. and Berzins, S.P. Immunol. 7:505-18 (2007; Margalit, M. and Ilan, Y. Liver Int. 25:501-4 (2005); Novak, J. et al. Int. Rev. Immunol. 26:49-72 (2007); Nowak, M. and Stein-Streilein, J. Int. Rev. Immunol. 26:95-119 (2007); Nikoopour, E. and Schwartz, J.A. Inflamm. Allergy Drug Targets 7:203-10 (2008)]. Induction of antigen-specific Treg may result in a long-lasting tolerance to β cell antigens, mediated by local immune modulation in the pancreatic draining lymph nodes (PLNs) [Homann, D. et al. J. Immunol. 163:1833-8 (1999); Homann, D. et al. Immunity 11:463-72 (1999)]. This intervention has shown great promise in animal models, but has had little efficacy in human trials. In the Diabetes Prevention Trial, only a sub-fraction of treated patients showed a beneficial effect with immunization with islet autoantigens [Skyler, J.S. et al. Diabetes Care 28:1068-76 (2005)]. Prevention of type 1 diabetes was only seen when patients were immunized during the pre-diabetic phase, and immunization was incapable of reverting recent-onset diabetes [Larche, M. and Wraith, D.C. Nat. Med. 11:S69-76 (2005)]. Therefore, antigen-specific interventions may require additional adjuvants in order to be used successfully in humans, especially in recent-onset diabetics [Harlan (2005) ibid.].

The invention showed a dose dependent effect on the intestine mucosal immune system, similar to that described in other systems of oral tolerance [Faria, A.M. and Weiner, H.L. Clin. Dev. Immunol. 13:143-57 (2006)]. As the effect on Treg distribution and insulin resistance parameters was different between mice administered high and low doses of the anti insulin antibodies, the data suggests that different dosing may determine the effect on subsets of Tregs promoted, and on the associated clinical effect.

In summary, the invention clearly demonstrate that antigen specific antibodies together with colostrum adjuvants can promote Tregs accumulation, and thereby serve as means for alleviating inflammatory response and improving Metabolic Syndrome complications. Further, according to the invention, Regulatory T lymphocytes in the adipose tissue and the SV can serve as a new therapeutic target in Metabolic Syndrome patients. Moreover, the immuneglobulins in the colostrum may promote regulatory T cells or any other cell related to the immune system in an antigen specific and non specific way, by targeting bystander antigens, or by being directed against non associated antigens.

Thus, the invention relates to an immuno-modulating composition for the treatment and prophylaxis of an immune-related disorder. More specifically, such composition comprises as an active ingredient bovine colostrum, and a bovine colostrum immunoglobulin preparation comprising anti-insulin antibodies, wherein the bovine colostrum immunoglobulin preparation is obtained from a bovine immunized with insulin. It should be noted that the colostrum-derived composition of the invention may further comprises any added adjuvant. The immunoglobulin preparation of the invention or any fragments thereof may be capable of recognizing and binding at least one antigen specific for the disorder and modulating immune-regulatory cells, specifically, regulatory T cells. Such modulation may results for example, in modulation of the Th1/Th2, Tr1/Th3 cell balance thereby activating or inhibiting an immune response specifically directed toward said disorder. Alternatively or additionally, the colostrums-derived immunoglobulin preparation of the invention or the immuno- modulatory composition derived therefrom, may act in an indirect manner by activation or promotion of specific subsets of regulatory cells, or antigen presenting cells, or by any type of cell-cell contact. Such immunoglobulin preparation may be directed towards different components of the immune-system. For example, activation of specific regulatory T cells, B cells or antigen presenting cells, or any other cells that associated with an effect on the immune system, or induces the secretion of cytokines or chemokines or affects the immune system in any other way. Alteration or promotion of immune cells may further involve induction of any type of regulatory cells, preferably, regulatory T cells, for example, Th3 cells, Tr1, T17 cells or any other type of regulatory, effector or suppressor cells. It should be noted that Th17 cells are a recently-identified subset of CD4⁺ T helper cells. They are found at the interfaces between the external environment and the internal environment, e.g., skin and lining of the GI tract. More specifically, it should be noted that the colostrum-derived immunoglobulin preparations of the invention may promote regulatory T cells or any other cell related to the immune system in an antigen specific and non specific manner, by targeting bystander antigens, or by being directed towards non associated antigens.

Thus, the immunoglobulin preparation may be antigen or disease specific or alternatively, may augment or induce specific cells or parts of the immune system in a non-antigen specific way, including an immune bystander effect.

It should be further appreciated that the immunoglobulin preparation of the invention may be used either for an active or a passive treatment.

According to one specific embodiment, the colostrum-derived immunoglobulin preparation may comprise monomeric, dimeric or multimeric immunoglobulin selected from the group consisting of IgG, IgA and IgM and any fragments thereof.

As indicated above, in ruminants, the principal compositional difference between colostrum and mature milk is the very high content of colostral immunoglobulin, of which IgG class makes up 80-90%, as demonstrated by Table 1.

**Table 1**

| **Ig mg/ml** | **Colostrum** | **Milk** |
|---|---|---|
| IgG total | 32-212 | 0.72 |
| IgG1 | 20-200 | 0.6 |
| IgG2 | 12.0 | 0.12 |
| IgA | 3.5 | 0.13 |
| IgM | 8.7 | 0.04 |

Thus, according to a specific embodiment, the colostrum-derived immunoglobulin preparation mainly comprises IgG, specifically, IgG1 and IgG2.

Immunoglobulin G (IgG) as used herein, is a multimeric immunoglobulin, built of two heavy chains γ and two light chains. Each complex has two antigen binding sites. This is the most abundant immunoglobulin and is approximately equally distributed in blood and in tissue liquids, constituting 75% of serum immunoglobulins in humans. In general, the number of IgG subclasses varied widely between different species, ranging from one subclass in rabbits to seven subclasses in horses, making it difficult to find orthologues. In humans, for example, IgG1 and IgG3 are the most pro-inflammatory IgG subclasses. In mice, however, IgG2a and IgG2b are the most pro-inflammatory IgG molecules showing a greater activity than mouse IgG1 and IgG3 in many *in vivo* model systems.

It should be noted that the family of Fc receptors (FcRs) for IgG (FcγRs) provides a prime example of how simultaneous triggering of activating and inhibitory signaling pathways sets thresholds for cell activation and thus generates a well-balanced immune response. Indeed, in a variety of human autoimmune disease, such as arthritis and systemic lupus erythematosus (SLE), aberrant expression or the presence of allelic variants of FcγRs with altered functionality have been observed that contribute to the pathogenesis of these diseases. Thus, the preparation of the immuno-modulatory composition of the invention that comprises mainly, IgG, may activate an FcγRs-mediated signaling pathway (either by the activating FcγRs or the inhibitory FcγRs).

Optionally or additionally, the immunoglobulin preparation may comprises a secretory antibody, specifically, sIgA.

Dimeric and multimeric IgA and IgM are secreted by a number of exocrine tissues. IgA is the predominant secretory immunoglobulin present in colostrum, saliva, tears, bronchial secretions, nasal mucosa, prostatic fluid, vaginal secretions, and mucous secretions from the small intestine. IgA output exceeds that of all other immunoglobulins, making it the major antibody produced by the body daily and is the major immunoglobulin found in human milk, whey and colostrum. IgM secretion is less abundant but can increase to compensate for deficiencies in IgA secretion. J chain containing IgA is produced and secreted by plasma B immunocytes located in the lamina propria just beneath the basement membrane of exocrine cells. IgA has a typical immunoglobulin four-chain structure (Mᵣ 160,000) made up of two heavy chains (Mᵣ 55,000) and two light chains (Mᵣ 23,000). In humans, there are two subclasses of IgA. These are IgA1 and IgA2 that have 1 and 2 heavy chains, respectively. IgA can occur as monomers, dimers, trimers or multimers. In plasma, 10% of the total IgA is polymeric while the remaining 90% is monomeric. The secreted IgA binds to a Mᵣ 100,000 poly-Ig receptor positioned in the basolateral surface of most mucosal cells. The receptor-IgA complex is next translocated to the apical surface where IgA is secreted. The binding of dimeric IgA to the poly-Ig receptor is completely dependent upon the presence of a J chain. Monomeric IgA will not bind to the receptor.

The difference in function of IgG and IgA, follows the position where the molecules operate. IgA is found mainly on mucosal surfaces where there is little in the way of tissue fluid to carry immune cells and chemicals. Therefore, IgA (often as a dimer) would be preferably used for physical neutralisation of pathogens, and may be too effective at other immune functions. IgGs are present in the tissue fluid and blood where there is the full collection of leukocytes, complement system, macrophages etc. may physically neutralize a pathogen effectively and are also more effective in a communication/presentation role than IgA, i.e., they tend to induce better opsonisation by phagocytes (e.g.' Killer T cells and macrophages) and switch on the complement system better.

More specifically, the immunoglobulin preparations may be obtained from any one of colostrum, colostrum serum, hyperimmunised milk or colostrum, colostrum whey (either cheese or casein), cheese or casein whey, directly from skim milk, whole milk, or a reconstituted form of such streams.

The colostrum
containing antigen-specific antibodies is preferably collected by milking the animal colostrum thus collected can either be used directly, may be further processed, for instance to purify antigen-specific antibodies. Methods for the (partial) purification of (antigen-specific) antibodies from colostrum or milk are present in the art and the following Example 1.

It should be further appreciated that any adjuvants may be added to the compositions of the invention. Appropriate adjuvants therefore may be any antigen, antibody, glycosphingolipids, proteins, cytokines, adhesion molecules, and component that can activate or alter the function of antigen presenting cell or of any other cell related to the immune system in a direct and indirect manner.

Alternatively, the immunoglobulin preparation may be an affinity purified antibody or any fragment thereof. The term "antibody" is meant to include both intact molecules as well as fragments thereof, such as, for example, Fab and F(ab')₂, which are capable of binding antigen. Fab and F(ab')₂ fragments lack the Fc fragment of intact antibody, clear more rapidly from the circulation, and may have less non- specific tissue binding than an intact antibody. It will be appreciated that Fab and F(ab')₂ and other fragments of the antibodies useful in the present invention may be used for immuno-modulation, according to the methods disclosed herein for intact antibody molecules. Such fragments are typically produced by proteolytic cleavage, using enzymes such as papain (to produce Fab fragments) or pepsin (to produce F(ab')₂ fragments).

An antibody is said to be "capable of specifically recognizing" a certain antigen if it is capable of specifically reacting with an antigen which is in this particular example an antigen or a mixture of antigens specific for a certain immune-related disorder, to thereby bind the molecule to the antibody.

An "antigen" is a molecule or a portion of a molecule capable of being bound by an antibody, which is additionally capable of inducing an animal to produce antibody capable of binding to an epitope of that antigen. An antigen may have one or more than one epitope. The term "epitope" is meant to refer to that portion of any molecule capable of being bound by an antibody that can also be recognized by that antibody. Epitopes or "antigenic determinants" usually consist of chemically active surface groupings of molecules such as amino acids or sugar side chains, and have specific three-dimensional structural characteristics as well as specific charge characteristics.

In yet another embodiment, the immunoglobulin preparation used as an active ingredient for the composition of the invention may be obtained from a non-human mammal immunized with at least one antigen or a mixture of at least two antigens specific for said disorder. According to one aspect, the antigen used for immunizing said non-human mammal, preferably, bovine, may be provided as any one of an isolated and purified peptide, a purified recombinant protein, a fusion protein, cell lysate, membranal preparation, nuclear preparation, or cytosolic preparation of any one of tissue culture cells, primary cells or tissue samples obtained from a subject suffering from said disorder.

According to another embodiment, the composition of the invention may optionally further comprise colostrum component/s such as for example, alarmins, defenensins, colostrinin, and any other colostrum or milk derived carbohydrates, glycolipids or any other molecules or components that may further enhance or inhibit modulation of an immune response, or any preparations, mixtures or combinations thereof. Moreover, the composition of the invention may comprise any additional adjuvant. Appropriate adjuvants therefore may be any antigen, antibody, glycosphingolipids, proteins, cytokines, adhesion molecules, and component that can activate or alter the function of antigen presenting cell or of any other cell related to the immune system in a direct and indirect manner.

It should be noted that the present disclosure further provides the use of colostrum or any colostrum-derived preparations for enhancing an immunomodulatory effect of an immunomodulatory therapeutic agent. Such colostrum-derived preparations may be therefore combined with any immunomodulatory therapeutic agent/s or any combination or mixture thereof, creating a combined immunomodulatory composition for the treatment and/or prevention of immune-related disorders, specifically, Metabolic Syndrome, autoimmune disorders, malignant and non-malignant proliferative disorder, genetic disease, infectious diseases and neurodegenerative disorders.

As indicated above, the composition of the disclosure is intended for preventing and/or treating an immune-related disorder, as used herein, the term "disorder" refers to a condition in which there is a disturbance of normal functioning. A "disease" is any abnormal condition of the body or mind that causes discomfort, dysfunction, or distress to the person affected or those in contact with the person. Sometimes the term is used broadly to include injuries, disabilities, syndromes, symptoms, deviant behaviors, and atypical variations of structure and function, while in other contexts these may be considered distinguishable categories. It should be noted that the terms "disease", "disorder", "condition" and "illness", are equally used herein. It should be further noted that an "immune-related disorder or disease" may be any disorder associated with, caused by, linked to, a non normal immune response. Such disorders may usually occur together with a disturbed immune response, or believed to have an impact on or by a non normal immune response.

According to one aspect, the immunoglobulin preparations comprised within the composition of the invention, recognize and bind at least one antigen specific for a disorder to be treated. Such recognition leads to alteration of regulatory T cells, and as a result, causes modulation of the Thl/Th2, Trl/Th3 cell balance either toward an anti-inflammatory Th2, Trl/Th3 immune response or toward a pro-inflammatory Th1 immune response. Thereby inhibiting or activating an immune response specifically directed toward said disorder.

It should be noted that any type of regulatory or effector cells, specifically regulatory T cells, including Th3 and Tr1 [T_{H}3, T cells are preferentially induced at mucosal surfaces and secrete transforming growth factor (TGF)-β] cells may be involved. Moreover, it should be noted that the colostrum-derived immunoglobulin preparations of the invention may promote regulatory T cells or any other cell related to the immune system in an antigen specific and non specific way, by targeting bystander antigens, or by being directed against non associated antigens.

According to one specific aspect, the immunoglobulin preparation recognizes and binds at least one antigen specific for said disorder and modulates immune-regulatory cells, specifically, regulatory T cells. Such modulation may results for example, in modulation of the Thl/Th2, Trl/Th3 cell balance toward an anti-inflammatory Th2, Tr1/Th3 immune response thereby inhibiting an immune response specifically directed toward said disorder. According to another alternative embodiment, the immunoglobulin preparation of the invention may be directed to antigens that are not specific to the treated disorder. Such antigens may be any target immune-related components having a modulatory effect on the immune-response. Thereby, recognition of such disease non-specific antigens by the immunoglobulin preparation of the invention may results in alteration of the immune-response. Such modulation may results for example, in modulation of the Th1/Th2, Trl/Th3 cell balance toward an anti-inflammatory Th2, Trl/Th3 immune response thereby inhibiting an immune response specifically directed toward said disorder.

According to one particular embodiment, the immunomodulatory composition of the invention may lead to a Th2, Trl/Th3 anti-inflammatory response. More specifically, such anti-inflammatory response may be accompanied by a decrease or reduction in the amount or expression of pro-inflammatory cytokines such as IL-2, IL-17, IL-23, IFN-γ, IL-6. Such decrease or reduction according to the invention may be a reduction of about 5% to 99%, specifically, a reduction of about 5%, 10%, 15%, 20%, 25%, 30%, 35%, 40%, 45%, 50%, 55%, 60%, 65%, 70%, 75%, 80%, 85%, 90%, 95% or 99% as compared to untreated control. In yet another specific embodiment, the composition of the invention may elevate and increase the amount or expression of anti-inflammatory cytokines such as TNF-β, IL-10, IL-4, IL-5, IL-9 and IL-13. More specifically, the increase, induction or elevation of the anti-inflammatory cytokines may be an increase of about 5%, 10%, 15%, 20%, 25%, 30%, 35%, 40%, 45%, 50%, 55%, 60%, 65%, 70%, 75%, 80%, 85%, 90%, 95% or 99% as compared to untreated control.

According to one aspect, such Th2, Tr1/Th3 response directing composition may be used for treating immune related disorder such as Metabolic Syndrom, graft rejection pathology, inflammatory disease and an autoimmune disease.

It should be appreciated that the anti-inflammatory effect of the immuno-modulatory composition of the invention may be achieved by activation or promotion of specific subsets of regulatory cells, antigen presenting cells or any type of cell-cell contact, or via direct or indirect activation of cytokines and/or chemokins. It should be further noted that any type of regulatory or effector cell, specifically regulatory T cells, including Th3 and Tr1 cells may be involved. Thus, the colostrum-derived immunoglobulin preparations of the invention may promote regulatory T cells or any other cell related to the immune system in an antigen specific and non specific way, by targeting bystander antigens, or by being directed against non associated antigens.

More specifically, an immune-related cell activated or promoted by the composition of the invention may be an APC (such as DC), Treg cell or any other cell associated directly on indirectly with the immune system including but not limited to platelets, macrophages, any type of B cell, T cell (including double negative cells), and any type of non-professional antigen presenting cell, adipocytes, endothelial cell, any type of cell that is part of an organ, specifically, an organ connected to the treated immune-related disorder and any type of cell having regulatory enhancing or suppressing properties. More particularly, the composition of the invention demonstrate immuno-modulation, specifically, either anti-inflammatory or pro-inflammatory effect on immune-related cells such as specific T regulatory cells for example, adipocytes and Antigen Presenting Cells (APC), such as DC. Therefore, according to one embodiment, the composition of the invention may be used for inducing at least one of T regulatory (Treg) cells, or any cell having regulatory properties, either suppressive or inductive, adipocyte and Antigen Presenting Cells (APC) in a subject suffering from an immune-related disorder.

According to one particular embodiment, an antigen specific for an immune-related disorder may be insulin. Accordingly, the colostrum-derived immunoglobulin preparation comprises anti-insulin antibodies. More particularly, as shown by the following Examples, the colostrum-derived anti-insulin antibodies of the invention clearly promote regulatory T cells accumulation, specifically, of CD4⁺CD25⁺Foxp3⁺, CD4⁺CD25⁺Foxp3⁺IL17⁺ and CD8⁺CD25 T regulatory cells in adipose tissue and in adipose tissue associated stromal vascular cells in a subject suffering of a Metabolic Syndrome or any of the conditions comprising the same.

Adipocytes are the cells that primarily compose adipose tissue, specialized in storing energy as fat. There are two types of adipose tissue, white adipose tissue (WAT) and brown adipose tissue (BAT), which are also known as white fat and brown fat, respectively, and comprise the two types of fat cells. White fat cells or monovacuolar cells contain a large lipid droplet surrounded by a layer of cytoplasm. The nucleus is flattened and located on the periphery. A typical fat cell is 0.1mm in diameter with some being twice that size and others half that size. The fat stored is in a semiliquid state, and is composed primarily of triglycerides and cholesteryl ester. White fat cells secrete resistin, adiponectin, and leptin. Brown fat cells or plurivacuolar cells are polygonal in shape. Unlike white fat cells, these cells have considerable cytoplasm, with lipid droplets scattered throughout. The nucleus is round, and, although eccentrically located, it is not in the periphery of the cell. The brown color comes from the large quantity of mitochondria.

As shown by the Examples, the composition of the invention significantly decreased the serum levels of triglycerides, ALT, AST and Glucose. Example 2 further shows that the composition of the invention leads to a significant increase in sensitivity to insulin. Therefore, according to one embodiment, the pharmaceutical composition of the invention leads to at least one of a decrease in the serum levels of cholesterol, triglycerides, ALT, AST and Glucose and an increase in the sensitivity to insulin or decrease in insulin resistance in a subject suffering of an immune-related disorder, for example, Metabolic syndrome. Wherein indicated decease, reduction, inhibition, it is meant that the composition of the invention leads to a reduction of about 5% to 99% of the serum level of any one of triglycerides, ALT, AST and Glucose, in a subject suffering of an-immune-related disorder. More specifically, such reduction may be a reduction of about, 5%, 10%, 15%, 20%, 25%, 30%, 35%, 40%, 45%, 50%, 55%, 60%, 65%, 70%, 75%, 80%, 85%, 90%, 95% and over 99%, as compared to the levels prior to the treatment, or the levels of untreated control. Wherein indicated increase, elevation, enhancement, induction, it is meant that the composition of the invention leads to induction, or increase of about 5% to 99% of the sensitivity to insulin in a subject suffering of an-immune-related disorder, such as metabolic syndrome. More specifically, such increase may be an increase of about, 5%, 10%, 15%, 20%, 25%, 30%, 35%, 40%, 45%, 50%, 55%, 60%, 65%, 70%, 75%, 80%, 85%, 90%, 95% and over 99%, of the sensitivity to insulin for example, as compared to the levels prior to the treatment, or the levels of untreated control.

According to another embodiment, the composition of the invention may further lead to a significant reduction in hepatic fat accumulation. Such reduction may be of about 5%-99% as compared to untreated control, as indicated above.

According to one specific aspect the composition of the invention may be used for preventing and/or treating autoimmune disease for example, Metabolic Syndrome or any of the conditions comprising the same, any condition associated with, caused by, linked to or believed to have an impact on metabolic syndrome, for example, at least one of dyslipoproteinemia (hypertriglyceridemia, hypercholesterolemia, low HDL-cholesterol), obesity, NIDDM (non-insulin dependent diabetes mellitus), IGT (impaired glucose tolerance), blood coagulability, blood fibrinolysis defects and hypertension.

The Metabolic Syndrome is characterized by a group of metabolic risk factors in one person including:
*Abdominal obesity (excessive fat tissue in and around the abdomen); *Atherogenic dyslipidemia (blood fat disorders - high triglycerides, low HDL cholesterol and high LDL cholesterol - that foster plaque buildups in artery walls); *Elevated blood pressure; *Insulin resistance or glucose intolerance; *Prothrombotic state (e.g., high fibrinogen or plasminogen activator inhibitor-1 in the blood); and *Proinflammatory state (e.g., elevated C-reactive protein in the blood). People with the metabolic syndrome are at increased risk of coronary heart disease and other diseases related to plaque buildups in artery walls (e.g., stroke and peripheral vascular disease) and type 2 diabetes.

More particularly, the composition of the disclosure is intended for the treatment of dyslipoproteinemia, which may include hypertriglyceridemia, hypercholesterolemia and low HDL-cholesterol, obesity, NIDDM (non-insulin dependent diabetes mellitus type 2), IGT (impaired glucose tolerance), blood coagulability, blood fibronolysis defects and hypertension.

According to one specific aspect
the immunomodulatory composition of the invention may be used for treating diabetes, particularly, Type 2 diabetes. Diabetes mellitus, often simply diabetes, is a syndrome characterized by disordered metabolism and inappropriately high blood sugar (hyperglycaemia) resulting from either low levels of the hormone insulin or from abnormal resistance to insulin's effects coupled with inadequate levels of insulin secretion to compensate. The characteristic symptoms are excessive urine production (polyuria), excessive thirst and increased fluid intake (polydipsia), and blurred vision. These symptoms are likely absent if the blood sugar is only mildly elevated.

The World Health Orgnization recogizes three main forms of diabetes mellitus: *Type 1, Type 2,* and *gestational diabetes* (occurring during pregnancy), which have different causes and population distributions. While, ultimately, all forms are due to the beta cells of the pancreas being unable to produce sufficient insulin to prevent hyperglycemia, the causes are different. Type 1 diabetes is usually due to autoimmune destruction of the pancreatic beta cells. Type 2 diabetes is characterized by insulin resistance in target tissues, this causes a need for abnormally high amounts of insulin and diabetes develops when the beta cells cannot meet this demand. Gestational diabetes is similar to type 2 diabetes in that it involves insulin resistance, hormones in pregnancy may cause insulin resistance in women genetically predisposed to developing this condition.

Acute complication of diabetes (hypoglycemia, ketoacidosis or nonketotic hyperosmolar coma) may occur if the disease is not adequately controlled. Serious long-term complications include cardiovascular disease (doubled risk), chronic renal failure, retinal damage (which can lead to blindness), nerve damage (of several kinds), and microvascular damage, which may cause impotence and poor healing. Poor healing of wounds, particularly of the feet, can lead to gangrene, which may require amputation.

According to another aspect, the immunomodulatory composition of the invention may be used for the treatment of Type 1 diabetes. Type 1 diabetes mellitus is characterized by loss of the insulin-producing beta cells of the islets of Langerhans in the pancreas, leading to a deficiency of insulin. The main cause of this beta cell loss is a T-cell mediated autoimmune attack.

The oral compositions described herein can be administered to a subject to treat or prevent disorders associated with an abnormal or unwanted immune response associated with cell, tissue or organ transplantation, e.g., renal, hepatic, and cardiac transplantation, e.g., graft versus host disease (GVHD), or to prevent allograft rejection.

According to a specifically preferred aspect, an autoimmune disease treated by the composition of the invention may be any one of rheumatoid arthritis, type 1 diabetes, type 2 diabetes, artherosclerosis, asthma, acute and chronic graft versus host disease, systemic lupus erythmatosus, scleroderma, multiple sclerosis, inflammatory bowel disease, psoriasis, uvietis, thyroiditis and immune mediated hepatitis.

According to another aspect, the composition of the invention may be used for the treatment of MS. Multiple Sclerosis (MS) is typically characterized clinically by recurrent or chronically progressive necrologic dysfunction, caused by lesions in the CNS. Pathologically, the lesions include multiple areas of demyelination affecting the brain, optic nerves, and spinal cord. The underlying etiology is uncertain, but MS is widely believed to be at least partly an autoimmune or immune-mediated disease.

Alternatively, the immunoglobulin preparation used by the composition of the invention may recognize and bind at least one antigen specific for the treated disorder and may modulates immune-regulatory cells, specifically, regulatory T cells. Such modulation may results for example, in modulation of the Th1/Th2 cell balance toward a pro-inflammatory Th1 immune response thereby activating an immune response specifically directed toward said disorder.

It should be appreciated that the pro-inflammatory effect of the immuno-modulatory composition of the invention may be achieved by activation or promotion of specific subsets of regulatory cells, antigen presenting cells or any type of cell-cell contact via direct or indirect activation, of cytokines, and/or chemokines.

According to this specific embodiment, modulation of the Th1/Th2, Th3 balance towards a pro-inflammatory Th1 response may be particularly applicable in immune related disorders having an undesired unbalanced anti-inflammatory Th2, Trl/Th3 response, for example, a malignant and non-malignant proliferative disorder, infectious disease, genetic disease and neurodegenerative disorders.

It should be appreciated that the non-human mammalian subject used for obtaining a colostrum-derived immunoglobulin preparation are farm animals. Means and methods of the invention are suited to obtain high and prolonged antigen-specific antibody production in the colostrum, milk or milk products of any lactating mammal. Preferably, said animal is a farm-animal. Farm animals are animals that are used on a commercial basis by man, be it for the production of milk, meat or even antibodies. Farm-animals already used for the commercial scale production of milk are preferred for the present invention since for these animals special lines and/or breeds exist that are optimized for milk production. Preferably, said farm-animal is a cow or a goat. More preferably said farm-animal is a cow. Any of the compositions of the invention are administered orally.

Orally administrated antibodies would be expected to be degraded in the gastrointestinal tract, given the low gastric pH and the presence of gastric and intestinal proteases. However, bovine colostral IgG (BCIg) has been cited as particularly resistant to GI destruction, relative to other immunoglobulins. Early studies of BCIg cited remarkable "resistance to proteolytic digestion in the intestine of a heterologous host". There is also evidence that bovine IgG1 is somewhat more resistant to proteolysis by trypsin, chymotrypsin and pepsin than other Igs. These results drove much of the early development of oral antibody therapy. More specifically, the composition of the invention may be suitable for mucosal administration, for example, pulmonary, buccal, nasal, intranasal, sublingual, rectal, vaginal administration and any combination thereof.

Moreover, the immunoglobulin preparation used by the compositions of the disclosure may be prepared in preparations such as food additives, aqueous solutions, oily preparations, emulsions, gels, etc., and these preparations may be administered orally, topically, rectally, nasally, bucally, or vaginally. The preparations may be administered in dosage formulations containing conventional non-toxic acceptable carriers and may also include one or more acceptable additives, including acceptable salts, polymers, solvents, buffers, excipients, bulling agents, diluents, excipients, suspending agents, lubricating agents, adjuvants, vehicles, delivery systems, emulsifiers, dis-integrants, absorbents, preservatives, surfactants, colorants, flavorants or sweeteners. An optional dosage form of the present invention may be a powder for incorporation into beverages, pills, syrup, capsules, tablets, granules, beads, chewable lozenges or food additives, using techniques known in the art. Thus, immuno-modulating composition of the invention may be administered in a form selected from the group consisting of orally-active powders, pills, capsules, teas, extracts, dried extracts, subliguals, sprays, dispersions, solutions, suspensions, emulsions, foams, syrups ,lotions, ointments, gels, pastes, dermal pathces, injectables, vaginal creams and suppositories.

Therapeutic formulations may be administered in any conventional dosage formulation. Formulations typically comprise at least one active ingredient, as defined above, together with one or more acceptable carriers thereof.

Each carrier should be both pharmaceutically and physiologically acceptable in the sense of being compatible with the other ingredients and not injurious to the patient. Formulations include those suitable for oral, administration. The formulations may conveniently be presented in unit dosage form and may be prepared by any methods well known in the art of pharmacy. The nature, availability and sources, and the administration of all such compounds including the effective amounts necessary to produce desirable effects in a subject are well known in the art and need not be further described herein.

The preparation of pharmaceutical compositions is well known in the art and has been described in many articles and textbooks, see e.g., Remington's Pharmaceutical Sciences, Gennaro A. R. ed., Mack Publishing Co., Easton, PA, 1990, and especially pp. 1521-1712 therein, fully incorporated herein by reference.

The pharmaceutical composition of the invention can be administered and dosed in accordance with good medical practice.

The composition of the invention may comprise the active substance in free form and be administered directly to the subject to be treated. Formulations typically comprise at least one active ingredient, as defined above, together with one or more acceptable carriers thereof. Each carrier should be both pharmaceutically and physiologically acceptable in the sense of being compatible with the other ingredients and not injurious to the patient.

Formulations include those suitable for oral administration. The formulations may conveniently be presented in unit dosage form and may be prepared by any methods well known in the art of pharmacy. The nature, availability and sources, and the administration of all such compounds including the effective amounts necessary to produce desirable effects in a subject are well known in the art and need not be further described herein.

The pharmaceutical compositions of the invention generally comprise a buffering agent, an agent that adjusts the osmolarity thereof, and optionally, one or more pharmaceutically acceptable carriers, excipients and/or additives as known in the art. Supplementary active ingredients can also be incorporated into the compositions. The carrier can be solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants.

As used herein "pharmaceutically acceptable carrier" includes any and all solvents, dispersion media, coatings, antibacterial and antifungal agents and the like. The use of such media and agents for pharmaceutical active substances is well known in the art. Except as any conventional media or agent is incompatible with the active ingredient, its use in the therapeutic composition is contemplated.

In instances in which oral administration is in the form of a tablet or capsule, the active drug components (immunoglobulin preparation) can be combined with a non-toxic pharmaceutically acceptable inert carrier such as lactose, starch, sucrose, glucose, modified sugars, modified starches, methylcellulose and its derivatives, dicalcium phosphate, calcium sulfate, mannitol, sorbitol, and other reducing and non-reducing sugars, magnesium stearate, stearic acid, sodium stearyl fumarate, glyceryl behenate, calcium stearate and the like. For oral administration in liquid form, the active drug components can be combined with non-toxic pharmaceutically acceptable inert carriers such as ethanol, glycerol, water and the like. When desired or required, suitable binders, lubricants, disintegrating agents and coloring and flavoring agents can also be incorporated into the mixture. Stabilizing agents such as antioxidants, propyl gallate, sodium ascorbate, citric acid, calcium metabisulphite, hydroquinone, and 7-hydroxycoumarin can also be added to stabilize the dosage forms. Other suitable compounds can include gelatin, sweeteners, natural and synthetic gums such as acacia, tragacanth, or alginates, carboxymethylcellulose, polyethylene, glycol, waxes and the like.

The immuno-modulatory effect of the composition of the invention may be achieved by activation or promotion of specific subsets of regulatory cells, antigen presenting cells, or any type of cell-cell contact via direct or indirect activation of cytokine/s and/or chemokines.

More particularly, the use of the composition of the invention may involve active use of the antibodies. For example, by activation of specific regulatory T cells, B cells, antigen presenting cells, or any type of effector or regulatory cells that is associated with an effect on the immune system, or inducing the secretion of cytokines or chemokines or affecting the immune system in any other way.

It should be noted that the immunoglobulin preparation used by the disclosure may be antigen or disease specific or alternatively, may target a disease non-specific component of the immune-system and thereby may augment or induce specific cells or parts of the immune system in a non antigen specific way, including an immune bystander effect, or non disease target antigens.

According to one aspect the invention further optionally uses further colostrum, milk or milk products component/s and any adjuvant/s, for the preparation of such composition.

According to another aspect, the colostrum-derived, milk or milk products-derived immunoglobulin preparation used by the invention comprises monomeric, dimeric and multimeric immunoglobulin selected from the group consisting of IgG, IgA and IgM and any fragments thereof. Preferably, the immunoglobulin preparation used by the invention mainly comprises IgG, specifically, IgG1 and/or IgG2. Alternatively, the immunoglobulin preparation used by the invention may comprise dimeric secretory IgA or any fragment thereof.

In yet another embodiment, the use according to the disclosure of colostrum-derived, milk or milk products-derived immunoglobulin preparation is for preparing a composition that optionally may further comprises colostrum, milk or milk products component/s and any adjuvant/s, preferably, alarmins, defenensins, colostrinin and any preparation, mixture or combination thereof. It should be further appreciated that the composition of the disclosure may comprise any additional adjuvant. Appropriate adjuvants therefore may be any antigen, antibody, glycosphingolipids, proteins, cytokines, adhesion molecules, and component that can activate or alter the function of antigen presenting cell or of any other cell related to the immune system in a direct and indirect manner. It should be noted that the present disclosure further provides the use of colostrum or any colostrum-derived preparations for enhancing an immunomodulatory effect of an immunomodulatory therapeutic agent.

The term alarmin, denotes an array of structurally diverse multifunctional host proteins that are rapidly released during infection or tissue damage, and that have mobilizing and activating effects on receptor-expressing cells engaged in host defence and tissue repair. Innate-immune mediators that have alarmin function include defensins, eosinophil-derived neurotoxin, cathelicidins and HMGB1.

Defensins are small (15-20 residue) cysteine-rich cationic proteins found in both vertebrates and invertebrates. They are active against bacteria, fungi and enveloped viruses. They consist of 15-20 amino acids including six to eight conserved cysteine residues. Cells of the immune system contain these peptides to assist in killing phagocytized bacteria, for example in neutrophil granulocytes and almost all epithelial cells. Most defensins function by penetrating the microbial's cell membrane by way of electrical attraction, and once embedded, forming a pore in the membrane which allows efflux.

The term "Colostrinin", as use herein refers to a polypeptide which, in its natural form, is obtained from mammalian colostrum. Colostrinin is sometimes known as "colostrinine", and has a molecular weight in the range 16,000 to 26,000 Daltons. Colostrinin may form a dimer or trimer of sub-units (each having a molecular weight in the range 5,000 to 10,000 Daltons, preferably 6,000 Daltons), and contains mostly praline (the amount of proline is greater than the amount of any other single amino acid).

Colostrinin is characterized in that it stimulates the production of cytokines, especially gamma interferon (IFN-γ), tumor necrosis factor (TNF-α), interleukins (e.g. IL-6 and IL-10) and various growth factors.

As indicated above, it should be noted that the immunoglobulin preparation used by the invention may be obtained from a non-human mammal, preferably a cow, immunized with at least one antigen or a mixture of at least two antigens specific for the disorder to be treated.

The disclosure thus provides the use of a colostrum-derived immuno-preparation for the preparation of an immuno-modulatory composition for the treatment and/or prevention of immune-related disorders.

According to one embodiment, the immunoglobulin preparation used by the disclosure recognizes and binds at least one antigen specific for a disorder to be treated. Alternatively, the immunoglobulin preparation of the invention may be directed to antigens that are not specific to the treated disorder. Such antigens may be any target immune-related components having a modulatory effect on the immune-response. The immunoglobulin of the invention modulates immune-regulatory cells, specifically, regulatory T cells. Such modulation may results for example, in modulation of the Th1/Th2, Trl/Th3 cell balance toward either an anti-inflammatory Th2, Trl/Th3 immune response or toward a pro-inflammatory Th1 immune response thereby inhibiting or activating an immune response specifically directed toward the disorder to be treated.

According to one specific aspect, the immunoglobulin preparation used by the invention recognizes and binds at least one antigen specific for the disorder to be treated and modulates immune-regulatory cells, specifically, regulatory T cells. In another embodiment, the immunoglobulin preparation of the disclosure may be directed to antigens that are not specific to the treated disorder. Such antigens may be any target immune-related components having a modulatory effect on the immune-response. Such modulation may results for example, in modulation of the Th1/Th2, Trl/Th3 cell balance toward an anti-inflammatory Th2, Trl/Th3 immune response thereby inhibiting an immune response specifically directed toward said disorder.

According to this aspect, an immune related disorder prevented and/or treated by such composition may be any one of Metabolic Syndrome or any of the conditions comprising the same, graft rejection pathology, inflammatory disease, an autoimmune disease and a non alcoholic fatty liver disease, hyperlipidemia and atherosclerosis.

According to one particular embodiment, an antigen specific for an immune-related disorder used by the invention may be insulin. Accordingly, the colostrum-derived immunoglobulin preparation comprises anti-insulin antibodies.

As shown by the following Examples, the anti insulin immunoglobulin preparations of the invention clearly promote regulatory T cells accumulation in adipose tissue and in adipose tissue associated stromal vascular cells, in a subject suffering of a Metabolic Syndrome or any of the conditions comprising the same.

According to one specific embodiment, the colostrum-derived anti-insulin antibodies of the invention lead to accumulation of CD4⁺CD25⁺Foxp3⁺, CD4⁺CD25⁺Foxp3⁺IL17⁺ and CD8⁺CD25 T regulatory cells in adipose tissue and in adipose tissue associated stromal vascular cells in a subject suffering of a Metabolic Syndrome or any of the conditions comprising the same.

According to another embodiment, the composition of the invention leads to at least one of a decrease in the serum levels of triglycerides, ALT, AST and Glucose and a decrease in insulin-resistance in a subject suffering of a Metabolic Syndrome or any of the conditions comprising the same. Therefore, a specific embodiment of the invention relates to the use of the immunoglobulin preparation in preparing a composition for the prevention and/or treatment and prophylaxis of Metabolic Syndrome or any of the conditions comprising the same, related to or caused by, more particularly, at least one condition of dyslipoproteinemia (hypertriglyceridemia, hypercholesterolemia, low HDL-cholesterol), obesity, NIDDM (non-insulin dependent diabetes mellitus, Type 2), IGT (impaired glucose tolerance), blood coagulability, blood fibrinolysis defects and hypertension.

According to one particular aspect, an autoimmune disease may be any one of rheumatoid arthritis, diabetes, asthma, acute and chronic graft versus host disease, systemic lupus erythmatosus, scleroderma, multiple sclerosis, inflammatory bowel disease and immune mediated hepatitis.

According to an alternative aspect, the immunoglobulin preparation used by the invention recognizes and binds at least one antigen specific for the disorder to be treated and modulates immune-regulatory cells, specifically, regulatory T cells. In another aspect, the immunoglobulin preparation of the invention may be directed to antigens that are not specific to the treated disorder. Such antigens may be any target immune-related components having a modulatory effect on the immune-response. Such modulation may results for example, in modulation of the Th1/Th2, Trl/Th3 cell balance toward a pro-inflammatory Th1 immune response. Activation of Th1 cells results in an immune response specifically directed toward the treated disorder. The antigen specific for an immune-related disorder used by the method of the invention may be insulin. Accordingly, the colostrum-derived immunoglobulin preparation comprises anti-insulin antibodies.

As shown by the following Examples, the anti insulin immunoglobulin preparations of the invention clearly promote regulatory T cells accumulation in adipose tissue and in adipose tissue associated stromal vascular cells, in a subject suffering of a Metabolic Syndrome or any of the conditions comprising the same.

According to one specific embodiment, the colostrum-derived anti-insulin antibodies used by the method of the invention lead to accumulation of CD4⁺CD25⁺Foxp3⁺, CD4⁺CD25⁺Foxp3⁺IL17⁺ and CD8⁺CD25 T regulatory cells in adipose tissue and in adipose tissue associated stromal vascular cells in a subject suffering of a Metabolic Syndrome or any of the conditions comprising the same. It should be noted that according to a specific embodiment, accumulation of these specific cell subsets in specific organs or tissues is meant that the number or the percentage or the cell population in the particular tissue is elevated or increased in about 5 to 95% as compared to untreated control. More specifically, such increase may be of about 5%, 10%, 15%, 20%, 25%, 30%, 35%, 40%, 45%, 50%, 55%, 60%, 65%, 70%, 75%, 80%, 85%, 90%, 95% or even 99%.

According to another embodiment, the composition used by the method of the invention leads to at least one of a decrease in the serum levels of triglycerides, ALT, AST and Glucose and a decrease in insulin-resistance in a subject suffering of a Metabolic Syndrome or any of the conditions comprising the same.

Thus, according to one specific embodiment, the method of the invention may be used for preventing and/or treating Metabolic Syndrome or any of the conditions comprising the same, for example, at least one of dyslipoproteinemia (hypertriglyceridemia, hypercholesterolemia, low HDL-cholesterol), obesity, NIDDM (non-insulin dependent diabetes mellitus), IGT (impaired glucose tolerance), blood coagulability, blood fibrinolysis defects and hypertension.

According to a specifically preferred embodiment, the method of the invention is specifically suitable for the treatment of a mammalian subject. "Mammal" or "mammalian" for purposes of treatment refers to any animal classified as a mammal including, human, research animals, domestic and farm animals, and zoo, sports, or pet animals, such as dogs, horses, cats, cows, etc. In a particular embodiment said mammalian subject is a human subject.

The terms "treat, treating, treatment" as used herein and in the claims mean ameliorating one or more clinical indicia of disease activity in a patient having an immune-related disease.

"Treatment" refers to therapeutic treatment. Those in need of treatment are mammalian subjects suffering from an immune-related disease. By "patient" or "subject in need" is meant any mammal for which administration of the immuno modulatory composition of the invention is desired, in order to prevent, overcome or slow down such infliction.

The terms "effective amount" or "sufficient amount" mean an amount necessary to achieve a selected result. The "effective treatment amount" is determined by the severity of the disease in conjunction with the preventive or therapeutic objectives, the route of administration and the patient's general condition (age, sex, weight and other considerations known to the attending physician).

As indicated above, generally, the dosage of needed to achieve a therapeutic effect will depend not only on such factors as the age, weight and sex of the patient and mode of administration, but also on the degree of disease progression and the potency of the particular derivative being utilized for the particular disorder of disease concerned.

It should be appreciated that the prevention or reduction of the risk of developing an immune-related disease is also encompassed within the scope of the invention. Such method may comprise the administration of a prophylactically effective amount of the composition of the invention or of the active ingredients comprised within such composition, to a person at risk of developing a disease.

The term "prophylactically effective amount" is intended to mean that amount of a pharmaceutical combined composition that will prevent or reduce the risk of occurrence of the biological or medical event that is sought to be prevented in a tissue, a system, animal or human by a researcher, veterinarian, medical doctor or other clinician.

It should be noted that for the method of treatment and prevention provided in the present invention, said therapeutic effective amount, or dosage, is dependent on severity and responsiveness of the disease state to be treated, with the course of treatment lasting from several days to several months, or until a cure is effected or a diminution of the disease state is achieved. Optimal dosing schedules can be calculated from measurements of drug accumulation in the body of the patient. Persons of ordinary skill can easily determine optimum dosages, dosing methodologies and repetition rates. In general, dosage is calculated according to body weight, and may be given once or more daily, weekly, monthly or yearly, or even once every 2 to 20 years. Persons of ordinary skill in the art can easily estimate repetition rates for dosing based on measured residence times and concentrations of the composition of the invention in bodily fluids or tissues. Following successful treatment, it may be desirable to have the patient undergo maintenance therapy to prevent the recurrence of the disease state, wherein the composition of the invention is administered in maintenance doses, once or more daily.

According to a further aspect, the invention provides an immuno-modulatory combined composition comprising as an active ingredient a combination or mixture of colostrum preparation and immunomodulatory therapeutic agent. The combined composition of the invention may be used for the treatment and/or prevention of immune-related disorders, specifically, Metabolic Syndrome, autoimmune disorders, malignant and non-malignant proliferative disorder, genetic disease, infectious diseases, and neurodegenerative disorders.

Throughout this specification and the Examples and claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

The following examples are representative of techniques employed by the inventors in carrying out aspects of the present invention.

### Examples

### Experimental procedures

### Cell lines:

- Hep 3B2.1-7 [Hep 3B; Hep-3B; Hep3B]- Human liver hepatocellular carcinoma cell line; ATCC number HB-8064™.
- HuH-7- Human liver hepatoma cell line; JCRB number JCRB0403.
- B-16 F-1- Mouse melanoma cell line; ATCC number CRL-6323™.
- Bcl-1- Mouse leukemia cell line; ATCC number CRL-1669.
- CCL-224-Colon cancer cells of human origin (colon, colorectal adenocarcinoma); ATCC number CCL-224™.
- PC-3-Prostate cancer- human origin (prostate, adenocarcinoma); ATCC number CRL-1435.
- LN-CaP- Prostate cancer, human origin (prostate, carcinoma); ATCC number CRL-1740™.

All cell lines are cultured in RPMI 1640 (BEIT HAEMEK - ISRAEL) supplemented with 100µg/ml penicillin, 100µg/ml streptomycin, 2mM L-glutamin and 10% FCS (BEIT HAEMEK - ISRAEL).

### Animals:

*Six to seven weeks old male C57BL/6-Leptin deficient mice (Ob/Ob mice, of the strain B6.V-Lep<ob>), purchased from The Jackson Laboratory, Bar Harbor, Maine, USA or from Harlan Laboratories (USA).
*Eleven weeks old female *psammomys obesus* desert sand rats obtained from Harlan Laboratories, Ein Karem, POB 12085, Jerusalem 91120, Israel (http://www.harlanisrael.com).
*Eleven to twelve old male Balb/C mice obtained from Harlan Laboratories, Ein Karem, POB 12085, Jerusalem 91120, Israel (http://www.harlanisrael.com).
*Eleven to twelve week old C57/bl mice obtained from Harlan Laboratories, Ein Karem, POB 12085, Jerusalem 91120, Israel (http://www.harlanisrael.com).

All animals are maintained in the Animal Core of the Hadassah-Hebrew University Medical School. Mice are administered standard laboratory chow and water *ad libitum,* and kept in 12-hour light/dark cycles. Animal experiments were carried out according to the guidelines of the Hebrew University-Hadassah Institutional Committee for Care and Use of Laboratory Animals, and with the committee's approval.

### Antibodies:

anti-CD4-Pacific Blue (eBioscience, San Diego, CA).
anti-CD8-FITC (eBioscience, San Diego, CA).
anti-IL-17-PE (eBioscience, San Diego, CA).
anti CD₂₅-PercP-Cy5.5 (eBioscience, San Diego, CA).
anti FOX3p PE-Cy725 (eBioscience, San Diego, CA).
anti-CD16/32 (eBioscience, San Diego, CA).

### Lysis Buffers:

Two lysis buffers are prepared, for the cytosolic fraction (buffer 1), and for the membranal fraction (buffer 2).
Buffer 1- 20mM Tris HCl-pH 7.5, 15mM NaCl, and 2mM EDTA.
Buffer 2- 50mM Tris HCl-pH 7.5, 150mM NaCl, 2mM EDTA, and 1% of NP-40 detergent (also called Igepal CA-630 by Sigma)

### Commercially used Ag:

*Insulin A (Insulin conjugated to KLH), batch no. 100908 (*Australia*).
*PreS1+ PreS for HBV obtained form VIROSTAT, (Immunochemicals for Infectious Disease Research, POB 8522, Portland, Maine 04104, USA). Pre-S1+S2 are recombinant antigen including amino acid residues from 1 to 174 of adw pre S1 region. The peptide is fused to terminal 6 histidines. Total molecular weight is 21 kDa.

*NS3, NS4a+b and NS5 for HCV, will be obtained from VIROSTAT (Immunochemicals for Infectious Disease Research, POB 8522, Portland, Maine 04104, USA). **NS3** is a recombinant antigen for HCV, comprising the C-terminal 380 amino acid peptide, and having a molecular weight of 68kDa. **NS4** is a recombinant antigen for HCV, NS4a+b is a peptide comprising amino acid residues from 1658 through 1863, and having a molecular weight of 19kDa. This peptide is fused beta-galactosidase peptide (114 kDa). Therefore, the total recombinant antigen has a molecular weight of 133kDa. **NS5** is a recombinant antigen for HCV and includes the NS5a region fused to GST. The total molecular weight of this antigen is 38 kDa. All recombinant antigens are expressed in *E. coli.*
*Toxin A for *Clostridium diffeicile* obtained from Sigma (Cat number C3977). The toxin is a lyophilized powder of molecular weight of about ∼270 kDa.
*Amyloid beta for Alzheimer disease: amyloid β protein (Aβ) is a synthetic peptide (1mg) which was purchased from Biosource International (Camarillo, California, USA).

### Preparation of Antigen - extraction of tissue culture:

Cytosolic fraction and membranal fraction are used as antigens for immunization.

### Preparation of cytosolic fraction:

Cytosolic fractionation is performed by rinsing the culture dish with 10-15ml of sterile PBS, lysing cells with 1-1.5ml of lysis buffer 1, scraping and collection of cells into 50ml eppendorff tubes on ice, dividing of the volume collected into 2ml eppendorff tubes, sonication of the cells (5 cycles, 25 seconds each cycle), followed by centrifugation (at 4°C, 14,000 RPM for 15 minutes), collection of supernatants, sampling (for protein quantification using the Bradford technique), and storage at -20°C.

### Preparation of membranal fraction:

The remaining pellet from the above mentioned centrifugation of the cytosolic fractionation is used for membranal fractionation performed by adding 100-250µl of lysis buffer 2, agitating tubes (30 minutes in 4°C), centrifuging (at 4°C, 14,000 RPM for 15 minutes), collecting supernatants, sampling (for protein quantification using the Bradford technique), and finally storing until use in -20°C.

### Preparation of Antigen - extraction of tissue:

Cytosolic fractions of the liver and pancreas of sacrificed animals are used for in the preparation of antigens for immunization (NASH model).

For the leukemia model, cyosolic and membranal fractions of spleen and peripheral blood cells are used in the preparation of antigens for immunization.

### Liver:

After harvesting the livers are transferred to ice cold PBS cut, minced and homogenized using a dounce homogenizer with 9ml of sterile cold lysis buffer 1, divided into eppendorff tubes (1.5ml in each tube), and kept on ice for 30 minutes, followed by sonication (five cycles of 25 seconds) and centrifugation (at 4°C, 14,000 RPM for 15 minutes). The supernatants are collected into one tube, sampled for protein quantification using the Bradford technique and stored at -20°C.

### Pancreas:

After harvesting the pancreases are transferred to ice cold PBS, cut, minced and homogenized using a dounce homogenizer with 2ml sterile cold lysis buffer 1, divided into eppendorff tubes (1ml in each tube), and kept on ice for 30 minutes, followed by sonication (five cycles of 25 seconds), and centrifugation (at 4°C, 14,000 RPM for 15 minutes). The supernatants are collected into one tube, sampled for protein quantification using the Bradford technique and stored at -20°C.

### Spleen:

Following excision the spleens are minced on cells dissociation grids (60 mesh) in RPMI 1640 medium, centrifuged (at 4°C, 1,400 RPM for 10 minutes) and the supernatant discarded; Red blood cells are lysed by adding 1ml of cold RBC lysis buffer (155mM ammonium chloride), followed by rinsing three times with cold PBS and centrifugation.

### Preparation of cytosolic fraction of spleen:

Cold buffer 1 was added to the pellet of spleen cells (in a 6:1 ratio of buffer to pellet) and the cells are divided into 2ml vials, kept on ice for 30 minutes, sonicated five times (25 seconds each time), and centrifuged (at 4°C, 14,000 RPM for 15 minutes); Supernatants are then collected from all vials, sampled for protein quantification, and kept at -20°C.

### Preparation of membranal fraction of spleen:

The remaining pellet from the above mentioned centrifugation step of the cytosolic fractionation is added with 100-250ml of buffer 2, agitated for 30 minutes at 4°C, and centrifuged (at 4°C, 14,000 RPM for 15 minutes). The supernatants are then collected from all vials, sampled for protein quantification and kept at -20°C.

### Peripheral blood cells:

Peripheral blood cells obtained from experimental animals are collected into EDTA containing vials, centrifuged (at 4°C, 1,400 RPM for 10 minutes), and the supernatant discarded; Red blood cells are lysed using RBC lysis buffer, followed by rinsing three times with cold PBS and centrifugation.

The following fractionation of the cytosol and membrane compartments is performed as previously described for the spleen.

### Isolation of Aortic valve lymphocytes

Aortic valve lymphocytes are isolated by crushing the valves through a stainless mesh (size 60, Sigma Chemical Co., St Louis, MO, USA). The cell suspension is placed in a 50-mL tube for 3 minutes and washed twice with cold PBS (1250 rpm for 10 minutes), and debris is removed. Cells are re-suspended in PBS, the cell suspension is strained through a nylon mesh presoaked in PBS, and unbound cells are collected. Cells are washed twice in 45 mL PBS. For aortic valve isolation, 20 mL of histopaque 1077 (Sigma Diagnostics, St Louis, MO, USA) is placed underneath cells suspended in 7 mL PBS in a 50-mL tube. The tube is centrifuged at 1640 rpm for 15 minutes at room temperature. Cells at the interface are collected, diluted in a 50-mL tube, and washed twice with ice-cold PBS (1250 rpm for 10 minutes).

### Isolation of splenic and hepatic lymphocytes for determination of T cell subpopulations:

Mice of different experimental models are sacrificed on day 60 of the experiment. Splenic lymphocytes and NKT cells are isolated and red blood cells removed. Intrahepatic lymphocytes are isolated as follows: After cutting the inferior vena cava above the diaphragm, the liver is flushed with cold PBS until it become pale, followed by removal of connective tissue and gall bladder. Livers and spleens were kept in RPMI-1640 + FCS. Then spleens were crushed through a 70µm nylon cell strainer (Falcon) and centrifuged (1250 rpm for 7 min) for the removal of cell debris. Red blood cells were lysed with 1ml of cold 155mM ammonium chloride lysis buffer and immediately centrifuged (1250 rpm for 3min). Splenocytes were then washed and resuspended with 1ml RPMI + FCS. Remains of connective tissue were removed. The viability by trypan blue staining was above 90%. For intrahepatic lymphocytes, livers were first crushed through a stainless mesh (size 60, Sigma) and the cell suspension was placed in a 50-ml tube for 5min to enable cell debris to descend. 10ml of Lymphoprep (Ficoll, Axis-Shield PoC AS, Oslo, Norway) was slowly placed under the same volume of cell suspension in 50-ml tubes. The tubes were then centrifuged at 1800 rpm for 18min. Cells in the interface were collected and moved to new tubes which were centrifuged again at 1800 rpm for 10min, to obtain a pellet of cells depleted of hepatocytes to a final volume of 250µl. Approximately 1x10⁶ cells/mouse liver, were recovered. Cells viability was detected by trypan blue staining.

### Isolation of adipocytes.

Adipose tissue (visceral fat pads) was minced and incubated in Krebs-Ringer bicarbonate buffer (3 mL/g adipose tissue) containing 10 mM glucose and 2.5% bovine serum albumin, incubated with 840 U/g collagenase type I (Sigma, Rehovot, Israel) at 37 °C with gentle agitation for 1 hour. Then filtered twice through chiffon mesh (100 µm) and centrifuged 50xg for 5 minutes. Floating adipocytes were then separated from the pellet of stromal vasculature (SV) fraction. The lower fraction was removed and centrifuged at 200xg for 5 min to pellet the SV cells. Cell number was then counted.

### Flow cytometery analysis (FACS) for determination of different subsets of lymphocytes

Following lymphocyte isolation from blood, spleen or any organ, triplicates of 2-5×10⁵ cells/500 µL PBS are placed in Falcon 2052 tubes, incubated with 4 mL of 1% BSA for 10 minutes, and centrifuged at 1400 rpm for 5 minutes. Cells are re-suspended in 10 µL FCS with 1:20 labeled (FITC, APC or PE-labeled) primary antibodies directed to the following lymphocyte markers: CD3, CD4, CD8, NK1.1, CD25, FOX p3, LAP cells, IL-17, Annexiin and surface markers for T cell activation. Cells-antibody mixtures are mixed every 10 minutes for 30 minutes. Cells are isolated using anti-CD3 and anti-CD4, anti-CD8, and anti-NK1.1, respectively. Cells are washed twice in 1% BSA and kept at 4°C until reading. For the control group, only 5 µL of 1% BSA are added. Surface staining was performed by incubating cells with antibodies and anti-CD16/32 (blocks Fc, eBioscience) at 4°C in FACS buffer containing PBS and 0.5% BSA, for 30 min. Cells were further washed twice with FACS buffer, resuspended in FACS buffer, and analyzed by flow cytometry. Analytical cell sorting is performed on 1×10⁴ cells from each group with a fluorescence-activated cell sorter (FACStar Plus, Becton Dickinson). Appropriate isotype controls were used in all experiments. Analysis was performed using a FACSCalibur instrument (Becton Dickinson, San Jose, CA). Only live cells were counted, and background fluorescence from non-antibody-treated lymphocytes was subtracted. Gates were set on forward- and side-scatters to exclude dead cells and red blood cells. Data was analyzed by the Consort 30 two-color contour plot (Becton Dickinson, Oxnard, CA, USA) or Cell Quest programs.

### FACS analysis for determination of NKT lymphocyte percentage

Immediately after lymphocyte isolation, triplicates of 2-5x10⁴ cells/500µl PBS are placed into Falcon 2052 tubes, incubated with 4 ml of 1% BSA for 10 minutes, and centrifuged at 350g for 5 minutes. For determination of the percentage of NKT lymphocytes, anti-CD3 and anti DX5 antibodies are used (Pharmingen, USA). Analytical cell sorting is performed on 1x10⁴ cells from each group with a fluorescence-activated cell sorter (FACSTAR plus, Becton Dickinson). Only live cells are counted, and background fluorescence from non-antibody-treated lymphocytes is subtracted. Gates are set on forward- and side-scatters to exclude dead cells and red blood cells. Data is analyzed with the Consort 30 two-color contour plot program (Becton Dickinson, Oxnard, CA), or the CELLQuest 25 program.

### Isolation of NKT lymphocytes and dendritic cells

Cell separation is performed using Magnetic Cell Sorting (MACS, Miltenyi Biotec, Germany) according to the manufacturer's instructions. Anti-CD3 and anti- DX5 magnetic beads are used for separation of NKT lymphocytes; anti-CD11c beads served for separation of dendritic cells. Beads are removed between the two steps according to the manufacturer's instructions. Above 95% accuracy is achieved by FACS analysis of cells.

### Measurement of serum cytokines

Serum IFNγ, IL10 and IL4 levels will be measured by a "sandwich" ELISA method using Genzyme Diagnostic kits (Genzyme Diagnostics, MA, USA), according to manufacturer's instructions.

### STAT protein expression

Expression of the transcription factors STAT (signal transducer and activator of transcription) 1, 4 and 6 and NFκB in splenocytes is determined by western blot analysis of splenocytes harvested from tested animals. Splenocytes (10X10⁶) are lysed in 100µl of lysis solution (Sigma). Proteins (100µg/lane) are resolved by electrophoresis on SDS-polyacrylamide (7.5%) gels, and electroblotted to nitrocellulose membranes (Schleicher & Schuell, Germany). Probing with a polyclonal rabbit antimouse antibody for the different tested STAT proteins and NFκB (Santa Cruz Biotechnology) is followed by addition of horseradish peroxidase-conjugated goat anti-rabbit IgG (Jackson Immuno Research, PA, and USA).

### Analysis of Metabolic Syndrom model animals

### Hepaotcellular Damage

Liver injury was evaluated by serum aspartic transaminase (AST) and alanine aminotransferase (ALT) activities, which were determined with an automatic analyzer.

### Measurements

The following parameters were measured in all mice every week: blood glucose, total cholesterol and triglyceride. Blood glucose values were measured with a standard glucometer. Plasma triglyceride and total cholesterol values were measured by a clinical chemistry analyzer Reflovet Plus machine (Roche Diagnostics, GmbH, Mannheim, Germany).

### Glucose Tolerance Test

Mice were subjected to a glucose tolerance test (GTT) on day 30 after overnight fasting. Glucose was administered orally (1.25 g per kg). Serum glucose measurements were performed on tail-vein blood every 15 minutes for 3 hours. Glucose levels were measured by a standard glucometer.

### Glucose Morning levels

Study groups were also evaluated by resting (non-fasting) morning glucose levels.

### Cytokine determination

IFN-γ and TGF-β levels were determined on serum by "sandwich" ELISA, using commercial kits (Quantikine, R&D Systems, Minneapolis, MN, USA). Serum insulin was also determined by "sandwich" ELISA, using the commercial kit of Mercodia AB (Uppsala, Sweden) according to the manufacturer's instructions.

### Statistics

Statistical significance was determined by unpaired, two-tailed Student's t test and only values of p < 0.05 are shown.

### MRI hepatic fat content measurement

Mice undergo magnetic resonance imaging (MRI) on day 60. Liver size is assessed, and hepatic fat content is measured by a double-echo chemical shift gradient-echo sequence technique, that provides in-phase and out-of-phase images in a single acquisition for assessment and quantification of liver fat. T1-weighted out-of-phase MR imaging is sensitive for detection of relatively small proportions of tissue fat. MR images are acquired by a 1.5-T system (Signa LX;GE, Milwaukee, USA). Double-echo MR imaging is performed with a repetition time (TR) of 125 msec, double echo times (TEs) of 4 and 6.5 msec, and a flip angle of 80°. Imaging parameters included section thickness of 3mm, a 13-cm field of view and a 256*160 matrix. Axial and coronal images are obtained. Signal intensity (SI) changes between in-phase and out of phase images are computed. The SI index is calculated as follows: SI index (SII) = (SIip-Siop)/SIip (SIip = in-phase SI; SIop=out of phase SI). Low SI index values indicate a smaller amount of tissue fat. An MRI fatty liver index (MFI = liver area*SII) is calculated for each animal.

### Triglyceride measurement

On day 60, serum triglyceride levels are measured using a spectrophotometer (Cobas DP-25P).

### Liver steatohepatitis score

A liver segment from each mouse was fixed in 10% formaldehyde and embedded in paraffin for histological analysis. Five sections (5 µm) are stained with hematoxylin/eosin and reviewed by two pathologists in a blinded fashion. Histological examination and the steatohepatitis grade scoring (NASH score) are performed using the steatohepatitis scoring system·

### Analysis of Leukemia model animals

### Assessment of GVHD

GVHD is assessed by the degree of weight loss, general appearance and a skin score based on a scale of cutaneous changes (including hair loss, skin desquamation and skin thickening) ranging from 0 (no GVHD) - 4 (maximal GVHD). Scale of cutaneous changes is assessed in five selected areas: forehead, periorbital, auricles, back and tail.

### MLR assay

Effector B10.D2 splenocytes (1x10⁶) from mice fed with Balb/c splenocytes, B10.D2 splenocytes, bovine serum albumin and regular chow is cultured in flat bottom microwell plates (Sterilin cat. n. M29ARTL) with irradiated (30 Gy) Balb/c or C57BL/6 spleen cells (2x10⁶) in a total volume of 0.2 ml RPMI 1640 culture medium, supplemented with 100µg/ml penicillin, 100 µg/ml streptomycin, 2 mM L-glutamine, with 5x10⁻⁵ 2M-ME and 10% FCS. After 72 hours in a 37⁰C humidified 5% CO2 incubator, 1µmCi of ³H TdR (5 Ci/nmol, Nuclear Research Center, Negev, Israel) is added to each well. Cells are collected 16 to 18 hours later on paper filters, using a multiple sample harvester (Titertek Cell Harvester 530; Flow Laboratories. McClean, VA 22102). Radioactivity is measured by a liquid scintillation counter. Background reactivity of B10.D2 splenocytes from the different groups against themselves is subtracted.

### Experimental analysis for immune-mediated hepatitis model

### Liver enzymes

Sera from individual mice are obtained. Serum AST and ALT levels are measured by an automatic analyzer as indicated for the Metabolic Syndrom animals.

### Histological Examination

Hematoxylin/eosin staining of paraffin-embedded liver sections is performed. Sections are examined by two experienced pathologists (VD, YS) that are blinded to the experiment conditions.

### Experimental analysis for melanoma model animals

### Proliferation assay

B16 melanoma cells are cultured in flat bottom microwell plates (Sterilin) in 3 concentrations: 50,000, 100.000 and 200.000 cells/well. Different concentrations of tested antibodies are added to appropriate wells, PBS is added to control wells. 5 hours later 1µmCi of 3H TdR (5 Ci/nmol, Nuclear Research Center, Negev, Israel) was added to all wells. After 24 hours in a 37⁰C, humidified 5% CO2 incubator, cells are collected on paper filters using a multiple sample harvester (Titertek Cell Harvester 530; Flow Laboratories. McClean, VA 22102). Radioactivity is measured by a liquid scintillation counter.

### Killing assay

B16 melanoma cells are cultured in flat bottom microwell plates (Sterilin) in 3 concentrations: 50,000, 100.000 and 200.000 cells/well. Different concentrations of tested antibodies are added to appropriate wells, PBS is added to control wells. After 24 hours in a 37⁰C humidified 5% CO2 incubator, cells are tripsynised and dyed with Tripan Blue. Dead cells are counted using light microscope.

### Apoptosis assay

Apoptosis is assayed using the Annexin-V-FLUOS Staining Kit. 1 x 10⁶ B16 melanoma cell pellet is resuspended in 100 µl of Annexin labeling solution and incubated for 20 minutes in room temperature. Cells are analyzed by flow cytometer.

### Experimental models:

*Metabolic syndrome- two models are used to examine the effect upon metabolic syndrome, the leptin deficiency model of Ob/Ob leptin-deficient mice and the diabetic Passamon model of the desert sand rat (*Psammomys obesus*). Mice are administered standard laboratory chow and water *ad libitum,* and kept in 12-hour light/dark cycles. Sand rats will be fed with high energy diet (Harlan Talked Global diet 2018SC+F) for eight weeks.
*Leukemia model- Balb/C male mice are administered standard laboratory chow and water *ad libitum,* kept in 12-hour light/dark cycles and injected with 1x10⁶ leukemia Bcl1 cells.
*Hepatocellular carcinoma- Balb/C male mice are administered standard laboratory chow and water *ad libitum,* and kept in 12-hour light/dark cycles. Mice are injected s.c. with 1x10⁷ Hep3b cells.
*Immune mediated hepatitis- Induction of Con A Hepatitis:
   C57/bl male mice are administered standard laboratory chow and water *ad libitum,* and kept in 12-hour light/dark cycles. Con A (Sigma) is dissolved in pyrogen-free PBS and injected into the tail vein at a dose of 500 µg/mouse (approximately 15 mg/kg).
*Melanoma- C57BL/6JOIaHsd mice are injected s.c. with 1 x 10⁶ B16-WT, B16-CD1d or B16-1C3 cells.

### Example 1

### Production of antibodies

Two dairy cows are immunized with a mixture of antigens. The antigens vaccine was administered during the last eight weeks of gestation. Colostral milk was collected during the first two days of lactation. The milk fat was removed and skim milk was pasteurized at 56° C for 30 minutes and then coagulated by renetting as in Hilpert, Human Milk Banking 1984. After removal of milk curd containing casein, the whey was centrifuged and the fine precipitate was discarded.

An equal volume of saturated ammonium sulfate solution was slowly added to the supernatant with continuous mixing as in Brandon et al. [Brandon et al., Aust. J. Exp. Biol. Med. Sci. 49:613 (1971)]. After centrifugation the resulting precipitate was saved and the supernatant containing lactose and salts was discarded.

The precipitate was dissolved in 0.01M TRIS-HCl buffer pH 8 containing 0.32M NaCl (30% of original volume). This solution was extensively dialyzed against five volumes of the same buffer using an Amicon spiral membrane SIY30 cartridge. The antibody solution was then concentrated to 10%, snap frozen and freeze dried.

### Production of antibody fragments from colostrum

Antibody fragments are prepared according to modified method based on the methods described by Jones R.G.A. and Landon J. [Jones R.G.A. and Landon J. J. Immunol. Methods 263: 57-74 (2002)]. Briefly, an equal volume of 0.2M Sodium Acetate buffer pH 4.0, is added to a colostrum pool obtained from immunized animals as described above. The pH of the diluted colostrum pool has been adjusted to 4.6 and incubated at 37°C for two hours to precipitate caseins. Subsequently, colostrum is centrifuged and filtered (0.45µm) to remove casein. The pH of the resultant colostral Whey has been adjusted to pH 4.0, followed by addition of Pepsin (Enzyme Solutions with 1:15,000 activity) at 5.0% w/w and incubation for twenty hours at 45°C. Pepsin digestion has been stopped by addition of 0.5 vol. of 1M Tris pH8 and cooling the reaction mix to 4°C. The pH of the reaction is adjusted to pH to 7.0 and the F(ab')2 mix is concentrated using 30kD ultra-filtration membrane and dia-filtrate vs. >50 volumes of 20mM sodium phosphate/150mM NaCl pH 6.0 buffer. Small peptides are then removed and the resulting solution containing F(ab')2, Pepsin and Large Peptides was then subjected to Q Sepharose Anion Exchange column that Binds Pepsin and acidic aggregates. To obtain purified F(ab')2, the remaining Fc and undigested Ig are removed from the F(ab')2 (mixed with remaining large Peptides and undigested Ig), by Protein G or by Prometic Mabsorbent A1P chromatography.

### Preparation of Fab' by 2-mercaptoethylamine (MEA)

To prepare Fab', 50ul (1/9 vol.) of 0.1M 2-mercaptoethylamine (MEA) in 0.1M sodium phosphate buffer pH 6.0, containing freshly prepared 5mM EDTA-disodium, are added to 0.1-3.0 mg of F(ab')2 in 0.45ml of 0.1 M sodium phosphate buffer, pH 6.0. The mixture is then incubated at 37°C for 90mins. Subsequently, the reaction mixture is applied on a PD-10 column, or a suitable G25 column, to remove the excess MEA. 0.1M sodium phosphate (pH 6.0, with 5mM EDTA-disodium) is used as the running buffer. The first protein peak which contains Fab', is collected and used for treating the corresponding different indications as indicated herein below.

### Preparation of colostrum containing anti-insulin antibodies for treating Metabolic syndrome

For preparation of each batch of colostrum powder, colostrum was collected from approximately 200 cows and frozen in individual bags for testing. For processing, colostrum was thawed, pooled and fat was removed. Each batch was pasteurized. Colostrum was concentrated by ultra-filtration to reduce volume before freeze drying. The ultra-filtration step reduced lactose in the final powder to less than 7% (from about 50%). Two freeze dried bovine-colostrum powders were used. These included colostrum derived solution (CDS) which contained insulin antibodies. This preparation was a mixture of colostrums originated from 3 cows which were vaccinated with Insulin A (Insulin conjugated to KLH), batch no. 100908. The colostrum powder was made up into a milky suspension by adding it to water.

### Example 2

### Immuno-modulatory effect of colostrum-derived anti-insulin antibodies on metabolic syndrome

To study the effect of the immunomodulatory composition of the invention upon metabolic disorders, the leptin deficiency model of leptin-deficient Ob/Ob mice was used. Leptin deficiency in rodents and humans results in a severe form of metabolic syndrome. No interventions aimed at correcting this disturbance have resulted in an amelioration of the syndrome. Recent evidence suggests that the immune system may play a pivotal role in the pathogenesis of the syndrome under conditions of leptin deficiency.

More specifically, to investigate the effect of colostrum-derived antibodies on immune-related disorders, the Ob/ob model was first examined by the inventors using insulin as a disease specific antigen related to metabolic syndrome. Therefore, the following Experimental groups were used:
Ob/Ob mice 7-8 weeks of age were divided into seven groups as detailed in Table 2. Mice were fed according to group allocation for 25 days and then sacrificed. Groups A and B were treated with colostrum derived solution (CDS) enriched with anti-insulin antibodies (anti-insulin specific, AIS-CDS). This preparation was a mixture of colostrums originated from 3 cows which were vaccinated with Insulin A (Insulin conjugated to KLH), batch no. 100908. The colostrum powder was made up into a milky suspension at low and high dosages [10-30 µg/ml (A) and 1000 µg/ml (B), w/v, respectively]. A purified (on protein G columns) CDS (purified anti insulin specific, PAIS-CDS) contained anti-insulin antibodies [2 grams of purified IgG from pooled colostrum (15 ml) from Insulin A immunized cows at high and low doses (groups C and D, respectively). Non anti insulin specific CDS (NAIS-CDS) prepared form freeze dried anti-ETEC hyper immune colostrum powder and contained anti E. coli antibodies in similar doses (groups E and F, respectively). Group G consisted of untreated controls which received PBS.

**Table 2 Experimental and control groups**

| **Group** | **Oral administered ligand** | **Dose** |
|---|---|---|
| **A** | Anti insulin specific CDS (AIS-CDS) | 1mcg |
| **B** | Anti insulin specific CDS (AIS-CDS)) | 1mg |
| **C** | Purified anti insulin specific CDS (PAIS-CDS) | 1mcg |
| **D** | Purified anti insulin specific CDS (PAIS-CDS) | 1 mg |
| **E** | Non anti insulin specific CDS (NAIS-CDS) | 1mcg |
| **F** | Non anti insulin specific CDS (NAIS) | 1 mg |
| **G** | Control | |

### Effect of oral administration of anti insulin antibodies on regulatory T cells

In order to determine whether feeding of antibodies can promote Tregs, the inventors examined the effect of oral administration of anti insulin colostrum derived antibodies (AIS-CDS) and purified anti-insulin antibodies (PAIS-CDS) on various subsets of regulatory T cells in mice. For this purpose, seven experimental groups as described above were fed according to the indicated diets. After 25 days the mice were sacrificed and lymphocytes were isolated from adipose and stromal vasculature (SV) tissues, livers and spleens. The distribution of the different subsets of regulatory T cells in the tissues was analyzed by FACTS. The results for groups B and D are presented in Table 3 as ratio versus control Group G.

**Table 3 Effect of oral administration of colostrum enriched with anti insulin antibodies on the tissue derived subsets of regulatory T cells**

| Group B | Tregs | Adipose tissue | SV | Liver | Spleen |
|---|---|---|---|---|---|
| | CD4+CD25+FoxP3+ | 1.38 | **2.15 | 0.73 | 13.60 |
| | CD4+CD25+FOXP3+ IL17+ | 2.73 | *1.52 | 1.38 | *2.34 |
| | CD8+CD25+ | 2.00 | 4.63 | 0.43 | 6.30 |
| | CD8+CD25+FOXP3+ | 1.08 | *1.77 | 2.10 | 6.47 |
| Group D | CD4+CD25+FoxP3+ | 1.38 | **1.26 | 0.77 | **40.00 |
| | CD4+CD25+FOXP3+ IL17+ | 1.77 | **3.44 | 2.43 | 0.51 |
| | CD8+CD25+ | **46.85 | **150.00 | **20.00 | **378.00 |
| | CD8+CD25+FOXP3+ | **1.02 | **1.76 | 7.67 | *3.42 |
| * - p≤ 0.05; **p≤0.01. | | | | | |

As shown in table 3, oral administration of colostrum enriched with anti-insulin antibodies in group B (AIS-CDS) or group D (PAIS-CDS) promoted in both cases CD4+CD25+Foxp3+ Tregs in the adipose tissue and in the stromal vasculature (SV). A marked increase was noted in the spleen, while a decrease in this population was observed in the liver of the two groups presented.

IL177 was suggested to play a role in the metabolic syndrome [Filippi, C.M. et al., Diabetes 57:2684-92 (2008)]. However, FoxP3+IL17+ cells have not been previously studied. An increase in a subset of CD4+CD25+FOXP3+IL17+ cells in the adipose tissue, SV and liver was noted in mice in both groups B and D, while this population decreased in the spleen of group D. The proportion of CD4+CD25+FOXP3+IL17+ lymphocytes as calculated by FACS in the adipose tissue versus the spleen was much higher in groups B and D versus G (5.46 and 16.14 vs. 4.68, for groups B, D, and G, respectively). The data supports an organ specific effect, for the accumulation of this subset of Tregs in the adipose tissue.

CD8+CD25+ and CD8+FoxP3+ cells were suggested to play a regulatory role in diabetes. As shown by Table 3, colostrum derived anti insulin antibodies (AIS) and purified anti insulin specific (PAIS-CDS) induced expansion of these two populations in all examined tissues of both groups B and D, except for in the liver of AIS fed mice of group B, in which a decrease in CD8+CD25+ cells was observed. In both groups B and D, an increase in CD8+CD25+FOXP3+ subsets of cells was noted in all four tested tissues.

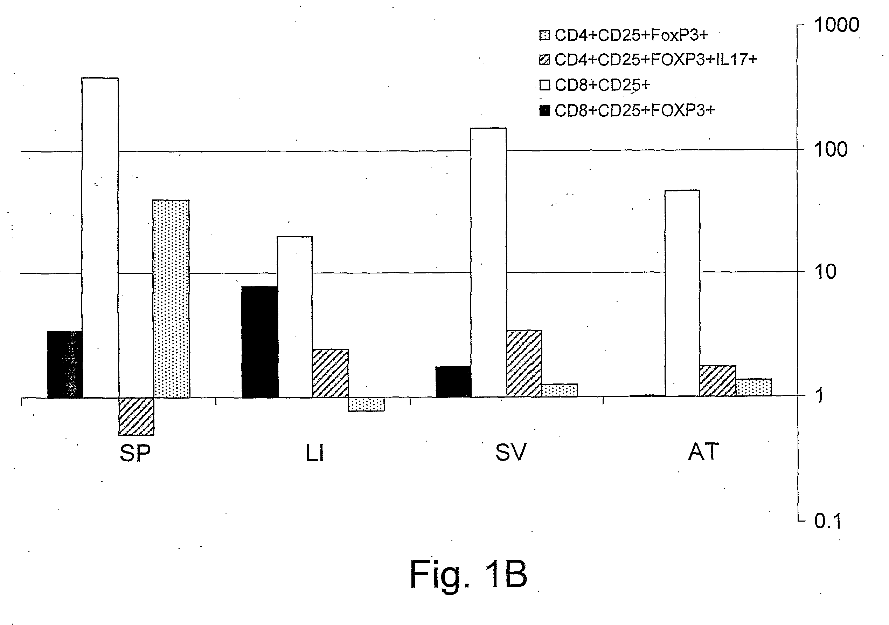

The results obtained from the FACS analysis that used for the ratio calculated and presented in Table 3 are summarized as bar charts in Figures 1A and 1B, presenting the alterations in the different subsets of Tregs in mice in groups B (1A) and D (1B) compared to mice in group G. An increase in all examined lymphocytes subsets, CD4+CD25+FOXP3+, CD4+CD25+FOXP3+IL17+, CD8+CD25+, and CD8+CD25+FOXP3+, is clearly observed in the adipose tissue and SV of mice in both groups. Taken together, these results indicate that feeding of anti insulin antibodies can promote Treg expansion in adipose tissue and stromal vasculature cells.

To determine the specificity of the increase in Tregs in the adipose tissue and SV, the proportion of these cells in each tissue versus the spleen was calculated as presented by Table 4.

**Table 4 Proportion of Tress in adipose tissue and stormal vasculature versus the spleen**

| **Group B** | **Tregs** | **Adipose tissue / spleen** | **Stromal vasculature / spleen** |
|---|---|---|---|
| | CD4+CD25+FoxP3+ | 0.10 | 0.16 |
| | CD4+CD25+FOXP3+IL17+ | 1.17 | 0.65 |
| | CD8+CD25+ | 0.32 | 0.74 |
| | CD8+CD25+FOXP3+ | 0.17 | 0.27 |
| | | | |
| **Group D** | CD 4+CD 2 5+FoxP 3+ | 0.03 | 0.03 |
| | CD4+CD25+FOXP3+IL17+ | 3.45 | 6.71 |
| | CD8+CD25+ | 0.12 | 0.40 |
| | CD8+CD25+FOXP3+ | 0.30 | 0.52 |

Table 4 shows that mice in group B, receiving AIS-CDS, had a higher proportion of CD4+CD25+Foxp3+ lymphocytes in the adipose tissue and SV as compared to group D. A significant increase was also seen in the proportion.of CD8+CD25+ cells. The data suggest that the adjuvant effect of the colostrum in group B has an effect on the distribution of Tregs leading to the accumulation of these two populations in the adipose tissue and SV.

### Effect of promotion of regulatory T cells in adipose tissue on the metabolic syndrome

To determine the effect of Tregs promotion on the metabolic syndrome, the inventors tested several parameters of insulin resistance and liver damage in mice subjected to the different diet regimes.

### Insulin resistance

Mice fed with anti insulin antibodies in low and high dose (groups A and B), mice fed with purified anti insulin antibodies (groups C and D), and untreated controls (group G) underwent glucose tolerance test every 15 days.

As shown in Figure 2A, both AIS-CDS (groups A, B) and PAIS-CDS (groups C, D) improved glucose tolerance demonstrated by lower glucose values at glucose tolerance test (p<0.01) with a decrease in the area under the curve (AUC) by more than 40%, as compared to the control group G (23962.5, 32589, 23818.7 and 23355 in groups A, B, C and D vs. 42448.7 in control group G).

### Insulin levels

To further assess the positive effect of the anti insulin antibodies, fasting serum insulin levels were tested following four weeks of feeding. The results presented in Figure 2B show that mice fed with AIS-CDS (groups A and B) or low dose of PAIS-CDS (group C) exhibited a decrease in serum insulin levels, indicating the beneficial impact of the anti insulin antibodies on insulin resistance. Moreover, the relatively profound decrease observed in group A provides data in support of an important role for the colostrum derived adjuvants in the metabolic effect.

### Serum glucose levels

In order to examine whether the fed antibodies can induce decrease in serum glucose levels, the mice of the different groups were tested for fasting serum glucose levels on a weekly basis. Figure 2C presents the decrease in mean fasting serum glucose levels. A high effect was noted for mice in groups A and B, orally administered with AIS-CDS, versus mice in groups C and D, fed with PAIS-CDS, or control mice in groups E and G.

To further examine the hypoglycemic effect of the anti insulin antibodies, the decrease in fasting serum glucose levels was analyzed on a weekly basis. Figure 2D shows the effect on the mean reduction of glucose levels per week for each experimental group. The highest glucose levels reduction is clearly demonstrated in groups A and B fed with AIS-CDS, compared to groups C and D, fed with PAIS-CDS and control groups E and G.

Taken together, the data obtained supports the importance of both the specificity of the antibodies, as well as the colostrum-derived adjuvants in the improvement of the metabolic syndrome.

### Hepatic triglyceride content and liver injury

Having shown that oral administration of anti insulin antibodies improves various metabolic syndrome markers, their effect on fat accumulation in the liver was tested at the end of the study. As clearly noted from Figure 3, oral administration of AIS-CDS in mice in groups A and B decreased hepatic triglyceride content compared to mice in groups C and D, fed with PAIS-CDS. Interestingly, a non specific antibody fed mice in group E also manifested an effect.

The inventors next evaluated whether the liver enzyme levels, which indicate liver injury, of animals fed with anti insulin antibodies have also improved due to the treatment. Therefore, ALT and AST serum levels of the different mice groups were measured at the end of the study. Promotion of adipose tissue and SV-derived Tregs, was accompanied by amelioration of the liver injury, manifested by a clear and significant decrease in ALT (450 vs. 750) and AST (570 vs. 770) serum levels vs. the control group G.

### Body and liver weights:

To assess whether the metabolic effect was independent from an effect on body weight, Figure 4A shows that among the study groups treated with AIS-CDS and PAIS-CDS, none of the groups differed significantly from the control group in neither the initial nor the final body and liver weights. As shown in Figure 4B, no changes in liver/body weight ratio were demonstrated in any of the groups.

### Example 3

### Immuno-modulation using the Metabolic Syndrome model

Encouraged by the beneficial effect of administration of colostrum-derived anti-insulin antibodies to the ob/ob model mice, the inventors searched for further disease-derived antigens in order to study the effect of the colostrum-derived compositions of the invention upon metabolic disorders. Therefore, both, the leptin deficiency model of leptin-deficient Ob/Ob mice and the diabetic Passamon model of desert sand rats (*Psammomys obesus*) are used.

The desert sand rat, a model of nutritionally-induced type II diabetes, develops severe metabolic disorders such as significant hyperinsulinemia, hyperglycemia and hypertriglyceridemia when fed on a high-energy diet.

### Preparation of Antigen:

The liver and pancreas of sacrificed mice and rats are used for the preparation of antigenic mixtures for immunizing cows for production of specific antibodies against antigens originated from metabolic syndrome models. After harvesting liver and pancreas are transferred and antigens are prepared for use in the creation of antibodies as described in experimental procedure. Antibodies specifically recognizing antigens prepared from animals of the Metabolic Syndrome model, are prepared and purified as described in Example 1 above.

### Use of specific antibodies in treatment of metabolic syndrome:

To examine the feasibility of using the colostrum-derived immunoglobulin preparation (antibodies) for treating Metabolic Syndrome Ob/Ob mice as well as diabetic desert rats, are orally administered using different concentrations and preparations of specific antibodies as compared to untreated controls. Animals are followed by glucose tolerance tests, determination of serum ALT and AST and triglyceride levels, assessment of liver size and hepatic fat content by magnetic resonance imaging (MRI) and histological examination as detailed in Experimental procedures.

### Example 4

### Activation of regulatory T cells by oral administration of antibodies

In order to examine the feasibility of using colostrum-derived antibodies for modulating different T regulatory cells, colostrum-derived *E.coli* specific antibodies were used. Therefore, ten mice (naive), age 11-12 weeks were fed for 7 days with PBS (A) or with colostrum enriched with antibodies to E Coli. (B), [100mg/ml, diluted in DDW and warmed for 1-2 hours at 37⁰C before administered to mice]. FACS analysis was preformed for different regulatory T cells. As presented by Tables 5 and 6, administration of colostrum enriched antibodies resulted in clear effect on several cell populations including CD45⁺LAP⁺ CD25⁺CD4⁺LAP⁻ and CD25-CD4⁺LAP⁺.

**Table 5 oral administration of hyper-immunized ETEC antibodies**

| **Fed** | | **CD3+ CD4-** | **CD3+ CD4+** | **CD25-CD4-** | **CD25-CD4+** | **CD25+ CD4+** | **CD45+ LAP+** | **CD3+ LAP+** | **CD25-CD4+ LAP-** | **CD25+ CD4+ LAP-** | **CD25+ CD4+ LAP+** | **CD25-CD4+ LAP+** |
|---|---|---|---|---|---|---|---|---|---|---|---|---|
| **PBS** | **A1** | 9.39 | 6.78 | 90.71 | 8.32 | 0.4 | 0.63 | 0.4 | 95.69 | 2.39 | 0 | 1.91 |
| | **A2** | 10.4 | 3.46 | 87.56 | 4.25 | 1.17 | 22.93 | 6.7 | 62.5 | 3.85 | 14.42 | 19.23 |
| | **A3** | 6.68 | 6.19 | 90.72 | 7.17 | 0.98 | 1.47 | 0.98 | 84.21 | 5.26 | 10.53 | 0 |
| | **A4** | 8.25 | 1.52 | 95.29 | 1.35 | 0.84 | 12.29 | 3.37 | 50 | 0 | 40 | 10 |
| | **A5** | 7.28 | 4.98 | 90.42 | 7.28 | 0.38 | 1.15 | 1.15 | 100 | 0 | 0 | 0 |
| | **Mean** | **8.4** | **4.586** | **90.94** | **5.674** | **0.754** | **7.694** | **2.52** | **78.48** | **2.3** | **12.99** | **6.228** |
| **Colostrum** | **B1** | 3.21 | 1.96 | 90.66 | 7.44 | 0.54 | 0.42 | 5.77 | 78.79 | 21.21 | 0 | 0 |
| | **B2** | 7.72 | 9.28 | 85.08 | 14.55 | 0.07 | 0.97 | 0.82 | 96.88 | 0.78 | 0 | 2.34 |
| | **B3** | 6.99 | 6.99 | 89.51 | 6.99 | 2.1 | 0.7 | 0 | 90.91 | 9.09 | 0 | 0 |
| | **B4** | 6.34 | 2.61 | 96.27 | 3.36 | 0 | 0.37 | 0.37 | 100 | 0 | 0 | 0 |
| | **B5** | 9.8 | 4.2 | 91.91 | 6.38 | 0.31 | 1.71 | 1.56 | 92.86 | 3.57 | 3.57 | 0 |
| | **Mean** | **6.812** | **5.008** | **90.686** | **7.744** | **0.604** | **0.834** | **1.704** | **91.888** | **6.93** | **0.714** | **0.468** |

**Table 6 oral administration of hyper-immunized ETEC antibodies**

| **Fed** | | **CD3+ CD8-** | **CD3+ CD8+** | **CD25-CD8-** | **CD25-CD8+** | **CD25+ CD8+** | **CD25+ CD8-** | **CD45+ LAP+** | **CD3+ LAP+** | **CD25-CD8+ LAP-** | **CD25+ CD8+ LAP-** | **CD25+ CD8+ LAP+** | **CD25-CD8+ LAP+** |
|---|---|---|---|---|---|---|---|---|---|---|---|---|---|
| **PBS** | **A1** | 12.58 | 6.1 | 91.55 | 7.3 | 0.02 | 1.13 | 0.36 | 0.14 | 99.21 | 0.39 | 0 | 0.39 |
| | **A2** | 6.44 | 3.8 | 93.56 | 4.95 | 0.17 | 1.32 | 0 | 0 | 95.65 | 4.35 | 0 | 0 |
| | **A3** | 7.99 | 4.23 | 93.1 | 6.11 | 0.31 | 0.47 | 0.78 | 0.47 | 96.3 | 0 | 3.7 | 0 |
| | **A4** | 6.06 | 3.56 | 94.23 | 4.04 | 0.19 | 1.54 | 1.15 | 0.87 | 91.89 | 0 | 0 | 8.11 |
| | **A5** | 11.33 | 2.83 | 89.52 | 2.83 | 1.13 | 6.52 | 17.85 | 3.68 | 70 | 0 | 30 | 0 |
| | **Mean** | **8.88** | **4.104** | **92.392** | **5.046** | **0.364** | **2.196** | **4.028** | **1.032** | **90.61** | **0.948** | **6.74** | **1.7** |
| **Colostrum** | **B1** | 5.36 | 2.18 | 91.55 | 6.98 | 0 | 1.47 | 1.14 | 0.71 | 95.65 | 0 | 0 | 4.35 |
| | **B2** | 9.83 | 4.01 | 86.55 | 6.01 | 1.34 | 6.11 | 32.06 | 4.2 | 79.07 | 0 | 9.3 | 11.63 |
| | **B3** | 7.27 | 3.64 | 93.03 | 4.24 | 0 | 2.73 | 1.52 | 0.3 | 100 | 0 | 0 | 0 |
| | **B4** | 6.7 | 1.73 | 95.78 | 3.14 | 0.19 | 0.89 | 1.03 | 0.42 | 97.5 | 2.5 | 0 | 0 |
| | **B5** | 9.93 | 3.16 | 93.32 | 4.6 | 0.72 | 1.35 | 7.67 | 3.88 | 80.58 | 0 | 13.89 | 5.56 |
| | **Mean** | **7.818** | **2.944** | **92**.**046** | **4.994** | **0.45** | **2.51** | **8.684** | **1**.**903** | **90.556** | **0**.**5** | **4**.**638** | **4**.**308** |

### Example 5

### Immuno-modulation using the Leukemia model

### Preparation of Antigen:

Eight to ten weeks old male Balb/C mice are I.V (tail vein) injected with a total volume of 0.2ml/mouse of 1 x 10⁶ Bcl 1 leukemic cells. White blood cells (WBC) are counted in blood samples taken every other day from the Plexus Orbital using a 20µl pipette. When WBC count reached a level of 1 x 10⁸/ml, mice are sacrificed and the spleen excised. After excision of spleens and the collection of peripheral blood cells cytosolic and membranal preparations are prepared for use as antigens in the creation of antibodies as described in Experimental Procedure. Antibodies specifically recognizing antigens prepared from animals of the leukemia model are prepared and purified as described in Example 1.

### Use of specific antibodies in the treatment of leukemia:

To examine the feasibility of using the colostrum-derived immunoglobulin preparation (antibodies) for treating leukemia, mice injected with Bcl1 leukemia cells, are orally administered using different concentrations and preparations of specific antibodies as compared to untreated controls. Animals of all tested groups are followed for 48 days for signs of chronic GVHD, leukemia progression and survival as detailed in Experimental procedures. Leukemia is assessed by white blood cell counts (WBC counts > 20 x 10⁹/L is considered leukemic) and spleen size. Tolerance induction in donor mice towards the minor histocompatibility antigens of recipient Balb/c mice is evaluated by one-way mixed lymphocyte reaction (MLR) tests as described in Experimental procedures.

### Example 6

### Immunomodulation using the Hepatocellular carcinoma model

### Preparation of Antigen:

Cytosolic and membranal fraction of HuH-7 and Hep3b cells are prepared and antigens for the creation of antibodies, are prepared as described in Experimental Procedure. Antibodies specifically recognizing antigens prepared from animals of the hepaotcellular carcinoma model are prepared and purified as described in Example 1.

### Use of specific antibodies in the treatment of hepatocellular carcinoma:

To examine the feasibility of using the colostrum-derived immunoglobulin preparation (antibodies) for treating Hepatocellular carcinoma (HCC), athymic C57/B1 mice sublethally irradiated and subcutaneously injected with 10 x 10⁶ trypsinized human Hep3B HCC, are used.

Mice treated with a specific antibody recognizing antigens obtained from hepaotcellular carcinoma models and control animals treated with nonspecific antibodies or with PBS are monitored for about 3-6 months. Biweekly measurements of tumor volume and body weight are performed and compared in all experimental groups.

In addition, FACS analysis for determination of CD4+, CD8+ and NKT lymphocyte subpopulations, as well as cytokine secretion assay (Levels of IFNγ, IL4, IL10, and IL12) measured by "sandwich" ELISA, and STAT proteins expression (STAT 1, 3, 4, 5, and 6) in splenocytes obtained from all experimental groups are measured as indicated in Experimental Procedures and compared to control animals.

### Example 7

### Immunomodulation using the immune mediated hepatitis model

Antibodies specifically recognizing antigens prepared from animals of the hepatitis model are prepared and purified as described in Example 1.

### Use of specific antibodies in the treatment of immune mediated hepatitis:

For immune mediated hepatitis model, eleven to twelve weeks old male C57/bl mice are tail vein injected with a dose of 500 µg/mouse (approximately 15 mg/kg) of Con A (MP Biomedicals, USA) which is dissolved in 50mM Tris pH 7, 150mM NaCl, 4mM CaCl₂, known to induce hepatitis. Animals of all tested groups are orally administered using different concentrations and preparations of specific antibodies, as compared to untreated controls. Animals of all tested groups are followed for the following parameters: serum aspartate aminotransferase (AST) and alanine aminotransferase (ALT) levels, histological examination of liver specimens, FACS analysis of intrahepatic and intrasplenic lymphocytes for NKT markers, measurement of serum cytokine levels and Western blot analysis for the expression of the transcription factors STAT 1, 4 and 6 and NFκB and are compared to control groups.

### Example 8

### Immunomodulation using the Melanoma model

### Preparation of Antigens:

Antigens for immunization are prepared from cytosolic and membranal fractions of B-16 cell line as described in Experimental procedures. Antibodies specifically recognizing antigens prepared from animals of the melanoma model are prepared and purified as described in Example 1.

### Use of specific antibodies in the treatment of melanoma:

The effect of the immuno-modulatory antibodies directed against melanoma-antigens is next examined using *in vitro* as well as *in vivo* models. Mice injected with three types of melanoma cell lines, the B16 melanoma, the Cd1d and the C13 melanoma, are used as the *in vivo* model, the B16 cell line is used as for the *in vitro* experiments.

The immunomodulatory effect of the colostrum-derived antibodies is first evaluated *in vitro* by proliferation assay, Killing assay and apoptosis as described in Experimental procedures.

The feasibility of using these antibodies for treating melanoma is next examined *in vivo* by comparing tumor formation in animals treated with the antibodies of the invention as compared to untreated controls. Tumor volume is calculated based on two measures of the two largest diameters, or number of metastasis in the lung. All experimental groups are further monitored for their effect on alteration of lymphocyte subset distribution, cytokine response, determination of NKT lymphocyte percentage and expression of STAT proteins as described in Experimental procedures.

### Example 9

### Immunomodulation using the Alzheimer disease model

### Preparation of Antigen:

Commercial amiloid beta is used as an antigen. Antibodies specifically recognizing the amyloid beta antigen are prepared and purified as described in Example 1.

### Use of specific antibodies in the treatment of Alzheimer:

To examine the feasibility of using the colostrum-derived immunoglobulin antibodies for treating Alzheimer disease (AD), an *in vivo* animal model is used. Transgenic mice expressing a mutation in the amyloid precursor protein (APP) and in the presenilin1 gene, is used as AD model by the inventors. Mice are treated with different concentrations of AD-specific colostrum-derived antibodies of the invention in different modes of delivery such as, drinking water, gavage, and i.p. injection, as compared to untreated controls. The treated animals are then evaluated for improvement in clinical parameters, including brain amyloid plaques formation.

### Example 10

### Immuno-modulation using the Colon cancer model

### Preparation of Antigen:

Cytosolic and membranal fractions of CCL-224 and HT29 are used for preparing antigens as described in Experimental procedures. Antibodies specifically recognizing colon carcinoma antigens are prepared and purified as described in Example 1.

### Use of specific antibodies in the treatment of colon cancer

To examine the feasibility of using the colostrum-derived antibodies of the invention for treating colon carcinoma, an *in vivo* tumor transplants in Athymic mice are used and tumor growth is analyzed. Balb-c/nude mice are injected with HT-29 cells in the right thigh and are treated with either powdered regular chow or different concentrations of anti-colon carcinoma colostrum-derive antibodies. Tumours are measured by calliper every other day. Mice are sacrificed three weeks after being inoculated with HT-29 cells, tumors are excised and weighed.

### Example 11

### Immuno-modulation using the Prostate cancer model

### Preparation of Ag:

Cytosolic and membranal fractions of pc-3, Du-145, LN-Cap, TSU-Pr1 cells, will be used for preparing antigens as described in Experimental procedures. Antibodies will be prepared and purified as described in Example 1.

### Use of specific antibodies in the treatment of prostate cancer:

To examine the feasibility of using the colostrum-derived antibodies for treating prostate cancer, mice injected with pc-3, Du-145, LN-Cap, TSU-Pr1 cells, are orally administered using different concentrations and preparations of specific antibodies as compared to untreated controls. Tumours are measured by calliper every other day. Mice are sacrificed three weeks after being inoculated with prostate cancer cells, tumors are excised, weighed and compared to untreated controls.

### Example 12

### Immuno-modulation using the Multiple sclerosis EAE model

### Preparation of A:

Antibodies to different cells, MBP, and cytokines are tested.

### Use of specific antibodies in the treatment of multiple sclerosis:

The feasibility of using the colostrum-derived antibodies of the invention in the treatment of autoimmune-diseases such as multiple sclerosis is analyzed using the Experimental autoimmune encephalomyelitis (EAE) model. EAE is an autoimmune inflammatory disease resulting in demyelination of the white matter in the CNS. In many of its clinical and histopathological aspects, EAE resembles human multiple sclerosis (MS) and acute disseminating encephalomyelitis. EAE can be induced in genetically susceptible animals by a single s.c. injection of myelin associated antigens, such as myelin oligodendrocyte glycoprotein (MOG), or proteolipid protein (PLP), emulsified in CFA and followed by a booster with Bordetella pertussis. A characteristic monophasic paralytic disease develops 10-13 days later. EAE serves as a useful experimental model for investigating new therapeutic strategies in MS. Two mouse models are used for analyzing the potential effect of the different colostrum-derived antibodies for the treatment of EAE, C57B1 mice immunized with MOG (myelin oligodendrocyte glycoprotein), and SJL mice immunized with PLP (proteolipid protein) are treated with different concentrations of the EAE specific antibodies as compared to untreated controls.

Clinical assessment of EAE is performed in all experimental groups according to the following criteria: 0, no disease; 1, decreased tail tone; 2, hindlimb weakness or partial paralysis; 3, complete hindlimb paralysis; 4, front and hindlimb paralysis; 5, moribund state.

### Example 13

### Immuno-modulation using the Asthma model

### Preparation of Ag:

Antibodies to various cytokines and glycolipids are prepared as described in Example 1.

### Use of specific antibodies in the treatment of asthma:

To examine the feasibility of using the colostrum-derived asthma-specific antibodies for treating asthma, the short and long term models of asthma are tested using the accepted parameters for follow up.

### Example 14

### Immuno-modulation using the Arthritis model

### Preparation of Ag:

Antibodies to collagen or to TNF are prepared as described in Example 1.

### Use of specific antibodies in the treatment of arthritis:

To examine the feasibility of using the colostrum-derived antibodies for treating arthritis, Adjuvant Arthritis (AA) is induced in Lewis rats by injection with 1 mg of Mycobacterium Tuberculosis H37Ra (Difco, Detroit, MI) in Complete Freund's Adjuvant (Difco) subcutaneously at the base of the tail. Rats are orally administered with different concentrations and preparations of the specific antibodies as compared to untreated controls. Severity of Arthritis (arthritis index) is assessed blindly as follows: 0 - no arthritis; 1 - redness of the joint; 2 - redness and swelling of the joint. The ankle and tarsal-metatarsal joints of each paw are scored. A maximum score of 16 can be obtained, but a score above 8 indicates a severe disease.

### Example 15

### Immuno-modulation using the Atherosclerosis model

### Preparation of Ag:

Antibodies to various cytokines, cells, LDL, different lipids are prepared according to Example 1.

### Use of specific antibodies in the treatment of atherosclerosis:

To examine the feasibility of using the colostrum-derived antibodies for treating atherosclerosis, ApoE deficient female mice fed a western diet (TD88137) from 8 weeks of age and Naive C57BL fed same diet, are orally administered using different concentrations and preparations of specific antibodies as compared to untreated controls. Animals of all tested groups are sacrificed after 18 weeks and parameters including body weight, plasma total cholesterol, triglycerides, HDL cholesterol (not pooled), serum cytokine levels, FACS analysis of CD4, CD8, NKT (CD3+NK1.1, CD4+NK1.1, CD8+NK1.1) in spleen and liver and examination of STAT1-6 and NFkβ expression in splenocytes, were compared to untreated controls. All tested groups are further monitored for atherosclerotic plaque lesion size (aortic sinus), atherosclerotic plaque distribution and Liver pathology. T cell proliferation response towards ox-LDL is also analyzed and compared to control untreated animals.

### Example 16

### Immuno-modulation using the Clostridum difficile-associated diarrhea model

### Preparation of Ag:

Toxin A for *Clostridium diffeicile* is obtained from Sigma (Cat number C3977). The toxin is a lyophilized powder of molecular weight of about ∼270 kDa. Antibodies are prepared and purified as described in Example 1.

## Claims

1. An immuno-modulating composition for use in the orally-administered treatment and/or prophylaxis of a Metabolic Syndrome disorder comprising as an active ingredient bovine colostrum and a bovine colostrum immunoglobulin preparation comprising anti-insulin antibodies, wherein the bovine colostrum immunoglobulin preparation is obtained from a bovine immunized with insulin and modulates regulatory T cells, thereby activating or inhibiting an immune response specifically directed toward said disorder.

2. The composition for use according to claim 1 further comprising bovine milk and any adjuvant/s.

3. The composition for use according to any one of claims 1 to 2, wherein said bovine colostrum immunoglobulin preparation comprises monomeric, dimeric or multimeric immunoglobulin selected from the group consisting of IgG, IgA and IgM and mixtures or combinations thereof and wherein said colostrum, or milk, comprises alarmins, defenensins, colostrinin or any preparations, mixtures and combinations thereof.

4. The composition for use according to any one of claims 1 to 3, wherein said immunoglobulin preparation modulates regulatory T cells leading to modulation of the Th1/Th2, Tr1/Th3 cell balance toward an anti-inflammatory Th2, Tr1/Th3 immune response or a pro-inflammatory Th1 immune response thereby inhibiting or activating an immune response specifically directed toward said disorder.

5. The composition for use according to any one of claims 1 to 3, wherein said immunoglobulin preparation modulates regulatory T cells leading to modulation of the Th1/Th2, Tr1/Th3 cell balance toward an anti-inflammatory Th2, Tr1/Th3 immune response thereby inhibiting an immune response specifically directed toward said disorder, and wherein said Metabolic Syndrome disorder is non alcoholic fatty liver disease, hyperlipidemia and atherosclerosis.

6. The composition for use according to claim 5, wherein said anti insulin immunoglobulin preparation promotes regulatory T cells accumulation in adipose tissue and in adipose tissue associated stromal vascular cells, in a subject suffering of a Metabolic Syndrome disorder.

7. The composition for use according to claim 6, wherein said composition leads to at least one of a decrease in the serum levels of triglycerides, ALT, AST and Glucose and a decrease in insulin-resistance in a subject suffering of a Metabolic Syndrome disorder.

8. Use of bovine colostrum and a bovine colostrum immunoglobulin preparation for preparing an immuno-modulating composition comprising anti-insulin antibodies for the orally-administered treatment and/or prophylaxis of a Metabolic Syndrome disorder, wherein said bovine colostrum immunoglobulin preparation is obtained from a bovine immunized with insulin and modulates regulatory T cells, thereby activating or inhibiting an immune response specifically directed toward said disorder.

9. The use according to claim 8, wherein said bovine colostrum immunoglobulin preparation comprises monomeric, dimeric or multimeric immunoglobulin selected from the group consisting of IgG, IgA and IgM and preparations, mixtures and combinations thereof and wherein said composition optionally further comprises bovine milk, any adjuvant/s and any mixture, preparation and combination thereof.

10. The use according to claim 9, wherein said immunoglobulin preparation modulates regulatory T cells leading to modulation of the Th1/Th2, Tr1/Th3 cell balance toward an anti-inflammatory Th2, Tr1/Th3 immune response or a pro-inflammatory Th1 immune response thereby inhibiting or activating an immune response specifically directed toward said disorder.

11. The use according to claim 10, wherein said immunoglobulin preparation modulates regulatory T cells leading to modulation of the Th1/Th2, Tr1/Th3 cell balance toward an anti-inflammatory Th2, Tr1/Th.3 immune response thereby inhibiting an immune response specifically directed toward said disorder, and wherein said Metabolic Syndrome disorder is any one of non alcoholic fatty liver disease, hyperlipidemia, and atherosclerosis.

12. The use according to claim 11, wherein said anti insulin immunoglobulin preparation promotes regulatory T cells accumulation in adipose tissue and in adipose tissue associated stromal vascular cells, in a subject suffering of a Metabolic Syndrome disorder.

13. The use according to claim 12, wherein said composition leads to at least one of a decrease in the serum levels of triglycerides, ALT, AST and Glucose and a decrease in insulin-resistance in a subject suffering of a Metabolic Syndrome disorder.

14. The composition and its use according to claim 1 or the use according to claim 8, wherein the Metabolic Syndrome disorder is dyslipoproteinemia, hypertriglyceridemia, hypercholesterolemia, low HDL-cholesterol, obesity, non-insulin dependent diabetes mellitus, impaired glucose tolerance, blood coagulability, blood fibrinolysis defects and hypertension.

## Patentansprüche

1. Immunmodulierende Zusammensetzung zur Anwendung in der oral verabreichten Behandlung und/oder Prophylaxe einer metabolischen Syndromerkrankung, die als Wirkstoff bovines Kolostrum und eine Immunglobulinzubereitung aus bovinem Kolostrum, umfassend anti-Insulin Antikörper, umfasst, wobei die Immunglobulinzubereitung aus bovinem Kolostrum von einem mit Insulin immunisierten Rind gewonnen wird und die regulatorischen T-Zellen moduliert, wodurch eine Immunantwort, die insbesondere gegen die Erkrankung gerichtet ist, aktiviert oder gehemmt wird.

2. Zusammensetzung zur Anwendung nach Anspruch 1, die ferner bovine Milch und ein Adjuvans bzw. Adjuvanzien umfasst.

3. Zusammensetzung zur Anwendung nach einem der Ansprüche 1 bis 2, wobei die Immunglobulinzubereitung aus bovinem Kolostrum monomeres, dimeres oder multimeres Immunglobulin, ausgewählt aus der Gruppe, bestehend aus IgG, IgA und IgM und Mischungen oder Kombinationen davon, umfasst und wobei das Kolostrum oder die Milch Alarmine, Defensine, Colostrinin oder Zubereitungen, Mischungen und Kombinationen davon umfasst.

4. Zusammensetzung für die Anwendung nach einem der Ansprüche 1 bis 3, wobei die Immunglobulinzubereitung regulatorische T-Zellen moduliert, was zu der Modulation der Th1/Th2-, Tr1/Th3-Zellbalance in Richtung einer entzündungshemmenden Th2-, Tr1/Th3-Immunantwort oder einer proinflammatorischen Th1-Immunantwort führt, wodurch eine Immunantwort, die insbesondere gegen die Erkrankung gerichtet ist, aktiviert oder gehemmt wird.

5. Zusammensetzung für die Anwendung nach einem der Ansprüche 1 bis 3, wobei die Immunglobulinzubereitung regulatorische T-Zellen moduliert, was zu der Modulation der Th1/Th2-, Tr1/Th3-Zellbalance in Richtung einer entzündungshemmenden Th2-, Tr1/Th3-Immunantwort führt, wodurch eine Immunantwort, die insbesondere gegen die Erkrankung gerichtet ist, gehemmt wird und wobei die metabolische Systemerkrankung eine nichtalkoholische Fettlebererkrankung, Hyperlipidämie und Atherosklerose ist.

6. Zusammensetzung für die Anwendung nach Anspruch 5, wobei die anti-Insulin Immunglobulinzubereitung die Akkumulation von regulatorischen T-Zellen im Fettgewebe und in mit dem Fettgewebe verbundenen stromal-vaskulären Zellen bei einem Individuum, das an einer metabolischen Syndromerkrankung leidet, fördert.

7. Zusammensetzung zur Anwendung nach Anspruch 6, wobei die Zusammensetzung mindestens zu einem von einer Senkung der Serumspiegel von Triglyceriden, ALT, AST und Glukose und einer Verringerung der Insulinresistenz bei einem Individuum, das an einer metabolischen Syndromerkrankung leidet, führt.

8. Anwendung von bovinem Kolostrum und einer Immunglobulinzubereitung aus bovinem Kolostrum zum Herstellen einer immunmodulierenden Zusammensetzung, umfassend anti-Insulin-Antikörper, für die oral verabreichte Behandlung und/oder Prophylaxe einer metabolischen Syndromerkrankung, wobei die Immunglobulinzubereitung aus bovinem Kolostrum von einem mit Insulin immunisiertem Rind gewonnen wird und die regulatorischen T-Zellen moduliert, wodurch eine Immunantwort, die insbesondere gegen die Erkrankung gerichtet ist, aktiviert oder gehemmt wird.

9. Anwendung nach Anspruch 8, wobei die Immunglobulinzubereitung aus bovinem Kolostrum monomeres, dimeres oder multimeres Immunglobulin, ausgewählt aus der Gruppe, bestehend aus IgG, IgA und IgM und Zubereitungen, Mischungen und Kombinationen davon, umfasst und wobei die Zusammensetzung gegebenenfalls ferner bovine Milch, ein Adjuvans bzw. Adjuvanzien und eine Mischung, Zubereitung und Kombination davon umfasst.

10. Anwendung nach Anspruch 9, wobei die Immunglobulinzubereitung regulatorische T moduliert, was zu der Modulation der Th1/Th2-, Tr1/Th3-Zellbalance in Richtung einer entzündungshemmenden Th2-, Tr1/Th3-Immunantwort oder einer proinflammatorischen Th1-Immunantwort führt, wodurch eine Immunantwort, die insbesondere gegen die Erkrankung gerichtet ist, aktiviert oder gehemmt wird.

11. Anwendung nach Anspruch 10, wobei die Immunglobulinzubereitung regulatorische T-Zellen moduliert, was zu der Modulation der Th1/Th2-, Tr1/Th3-Zellbalance in Richtung einer entzündungshemmenden Th2-, Tr1/Th3-Immunantwort führt, wodurch eine Immunantwort, die insbesondere gegen die Erkrankung gerichtet ist, gehemmt wird, und wobei die metabolische Syndromerkrankung eine von einer nichtalkoholischen Fettlebererkrankung, Hyperlipidämie und Atherosklerose ist.

12. Anwendung nach Anspruch 11, wobei die anti-Insulin Immunglobulinzubereitung die Akkumulation von regulatorischen T-Zellen im Fettgewebe und in mit dem Fettgewebe verbundenen stromal-vaskulären Zellen bei einem Individuum, das Leiden an einer metabolischen Syndromerkrankung leidet, fördert.

13. Anwendung nach Anspruch 12, wobei die Zusammensetzung mindestens zu einem von einer Senkung der Serumspiegel von Triglyceriden, ALT, AST und Glukose und einer Verringerung der Insulinresistenz bei einem Individuum, das an einer metabolischen Syndromerkrankung leidet, führt.

14. Zusammensetzung und deren Anwendung nach Anspruch 1 oder Anwendung nach Anspruch 8, wobei die metabolische Syndromerkrankung Dyslipoproteinämie, Hypertriglyceridämie, Hypercholesterinämie, niedriges HDL-Cholesterin, Übergewicht, nicht-insulinabhängiger Diabetes mellitus, gestörte Glukosetoleranz, Gerinnbarkeit des Blutes, defekte Fibrinolyse des Bluts und Bluthochdruck ist.

## Revendications

1. Composition immunomodulatrice à utiliser dans le traitement administré par voie orale et/ ou la prophylaxie d'un trouble du syndrome métabolique comprenant comme ingrédient actif du colostrum bovin et une préparation d'immunoglobuline de colostrum bovin comprenant des anticorps anti-insuline, dans laquelle la préparation d'immunoglobuline de colostrum bovin est obtenue à partir d'un bovin immunisé avec de l'insuline et module des cellules T régulatrices, activant ou inhibant ainsi une réponse immunitaire spécifiquement adressée vers ledit trouble.

2. Composition à utiliser selon la revendication 1, comprenant en outre du lait bovin et tout adjuvant.

3. Composition à utiliser selon l'une quelconque des revendications 1 à 2, dans laquelle ladite préparation d'immunoglobuline de colostrum bovin comprend une immunoglobuline monomère, dimère ou multimère choisie dans le groupe constitué d'IgG, IgA et IgM et des mélanges ou des combinaisons de ces derniers et dans laquelle ledit colostrum ou lait comprend des alarmines, des défensines, de la colostrinine ou toute préparation, mélange et combinaison de ces dernières.

4. Composition à utiliser selon l'une quelconque des revendications 1 à 3, dans laquelle ladite préparation d'immunoglobulines module les cellules T régulatrices conduisant à la modulation de l'équilibre des cellules Th1/ Th2, Tr1/ Th3 vers une réponse immunitaire Th2, Tr1/ Th3 anti-inflammatoire ou une réponse immunitaire Th1 pro-inflammatoire inhibant ainsi ou activant une réponse immunitaire spécifiquement dirigée vers ledit trouble.

5. Composition à utiliser selon l'une quelconque des revendications 1 à 3, dans laquelle ladite préparation d'immunoglobulines module les cellules T régulatrices conduisant à la modulation de l'équilibre des cellules Th1/ Th2, Tr1/ Th3 vers une réponse immunitaire Th2, Tr1/ Th3 anti-inflammatoire inhibant ainsi une réponse immunitaire spécifiquement adressée vers ledit trouble et dans laquelle ledit trouble du syndrome métabolique est une stéatose hépatique non alcoolique, une hyperlipidémie et une athérosclérose.

6. Composition à utiliser selon la revendication 5, dans laquelle ladite préparation d'immunoglobuline anti-insuline favorise l'accumulation de cellules T régulatrices dans le tissu adipeux et dans des cellules vasculaires stromales associées au tissu adipeux, chez un sujet souffrant d'un trouble du syndrome métabolique.

7. Composition à utiliser selon la revendication 6, dans laquelle ladite composition conduit, au moins, à une diminution des taux sériques de triglycérides, ALT, AST et glucose et une diminution de la résistance à l'insuline chez un sujet souffrant d'un trouble du syndrome métabolique.

8. Utilisation de colostrum bovin et d'une préparation d'immunoglobuline de colostrum bovin pour préparer une composition immunomodulatrice comprenant des anticorps anti-insuline pour le traitement administré par voie orale et/ ou la prophylaxie d'un trouble du syndrome métabolique, ladite préparation d'immunoglobuline de colostrum bovin étant obtenue à partir d'un bovin immunisé avec de l'insuline et qui module les cellules T régulatrices, activant ou inhibant ainsi une réponse immunitaire spécifiquement dirigée vers ledit trouble.

9. Utilisation selon la revendication 8, dans laquelle ladite préparation d'immunoglobuline de colostrum bovin comprend une immunoglobuline monomère, dimère ou multimère choisie dans le groupe constitué d'IgG, IgA et IgM et des préparations, mélanges et combinaisons de ces derniers et dans laquelle ladite composition comprend, de manière facultative, du lait bovin, tout adjuvant et tout mélange, préparation et combinaison de ces derniers.

10. Utilisation selon la revendication 9, dans laquelle ladite préparation d'immunoglobuline module les cellules T régulatrices conduisant à la modulation de l'équilibre des cellules Th1/ Th2, Tr1/ Th3 vers une réponse immunitaire Th2, Tr1/ Th3 anti-inflammatoire ou une réponse immunitaire Th1 pro-inflammatoire inhibant ainsi ou activant une réponse immunitaire spécifiquement adressée vers ledit trouble.

11. Utilisation conformément à la revendication 10, dans laquelle ladite préparation d'immunoglobuline module les cellules T régulatrices conduisant à la modulation de l'équilibre des cellules Th1/ Th2, Tr1/ Th3 vers une réponse immunitaire Th2, Tr1/ Th3 anti-inflammatoire inhibant ainsi une réponse immunitaire spécifiquement adressée vers ledit trouble et dans laquelle ledit trouble du syndrome métabolique est une stéatose hépatique non alcoolique, une hyperlipidémie et une athérosclérose.

12. Utilisation selon la revendication 11, dans laquelle ladite préparation d'immunoglobuline anti-insuline favorise l'accumulation de cellules T régulatrices dans le tissu adipeux et dans des cellules vasculaires stromales associées au tissu adipeux, chez un sujet souffrant d'un trouble du syndrome métabolique.

13. Utilisation selon la revendication 12, dans laquelle ladite composition conduit, au moins, à une diminution des taux sériques de triglycérides, ALT, AST et glucose et une diminution de la résistance à l'insuline chez un sujet souffrant d'un trouble du syndrome métabolique.

14. Composition et son utilisation selon la revendication 1 ou l'utilisation selon la revendication 8, dans laquelle le trouble du syndrome métabolique est une dyslipoprotéinémie, une hypertriglycéridémie, une hypercholestérolémie, un faible cholestérol HDL, une obésité, un diabète sucré non insulinodépendant, une tolérance au glucose altéré, une coagulabilité du sang, des défauts de la fibrinolyse sanguine et une hypertension.
